# EUROPEAN PATENT SPECIFICATION

(11) **EP 1 613 361 B1**
(45) Date of publication and mention of the grant of the patent: **22.08.2007**
(21) Application number: 04750233.1
(22) Date of filing: 16.04.2004
(51) Int. Cl.: A61L 9/02, A61L 9/03

(54) **METHOD AND DEVICE FOR IMPROVED SCENT DELIVERY**
VERFAHREN UND VORRICHTUNG FÜR VERBESSERTE DUFTABGABE
PROCEDE ET DISPOSITIF DE DIFFUSION DE PARFUMS AMELIOREE

(30) Priority: 16.04.2003 US 417456; 16.04.2003 US 417462; 01.10.2003 US 507807 P; 01.10.2003 US 507772 P
(43) Date of publication of application: 11.01.2006
(60) Divisional application: 06126779.5
(73) Proprietor: THE PROCTER & GAMBLE COMPANY, Cincinnati, Ohio 45202 (US)
(72) Inventor: WOO, Ricky, Ah-Man, Hamilton, OH 45011 (US); ALONSO, Mario, Loveland, OH 45140 (US); HECT, John, Phillip, West Chester, OH 45069 (US); REECE, Steven, Hamilton, OH 45013 (US); KVIETOK, Frank, Andrej, Aurora, CO 800016 (US); ST. PIERRE, Eileen, Marie, Cincinnati, OH 45226 (US); READNOUR, Christine, Marie, Ft. Mitchell, KY 41017 (US); KAISER, Carl, Eric, Mason, OH 45040 (US); BAILLELY, Susan, Mason, OH 45040 (US); AGAMI, Sion, Mason, OH 45040 (US); LIU, Zaiyou, West Chester, OH 45096 (US)
(74) Representative: Howard, Phillip Jan
(86) International application number: PCT/US2004/011838
(87) International publication number: WO 2004/093929

(56) References cited:
- EP-A- 1 260 255
- GB-A- 2 279 010
- US-A- 4 629 604
- US-A- 5 727 186
- US-B1- 6 465 420

## Description

### Field of the Invention

The present invention relates to a method, a device and a system for improved scent delivery. The invention delivers a scent experience to the user, which diminishes less over a period of time, as compared with other methods, devices and systems. The invention thus provides the user with a scent experience that is more noticeable for a longer period.

### Background of the Invention

It is generally known to use an electrical device to evaporate a perfume and/or fragrance composition into a space, particularly a domestic space, e.g., a living room, to provide a pleasant aroma. There are a variety of such devices on sale, including for example the AIRWICK® Diffuser ACTIF® (manufactured by Reckitt Benckiser) or the AMBI-PUR® fragrance diffuser (manufactured by Sara Lee). Generally, these devices consist of a perfume or fragrance source, an electrical heater, and a power supply. By the application of heat to the perfume or fragrance source, there will be a continuous supply of the perfume or fragrance to the space in which the device is placed.

The problem with this arrangement is that a person occupying the space will quickly become accustomed to the perfume or fragrance and, after a while, will not perceive the fragrance strength as being as intense, and may not notice it at all. This is a well-known phenomenon called habituation. A solution to this problem has been sought

One effort to deal with this problem is described in U.S. Patent Application Publication No. US 2002/0159916 A1, to Whitby et al. The Whitby et al. patent application discloses a method and device adapted to provide to a space, two or more fragrance compositions, at least one of which fragrance compositions is provided periodically. The method and device may provide a continuous supply of a first fragrance composition and a periodic supply of a second fragrance composition. The fragrance composition(s) may be vaporized by heating and may include deodorant and/or insecticidal compounds. The fragrance compositions are preferably chosen such that the two fragrance compositions contrast with one another or have different notes. The fragrance composition is generally pulsed from a device which includes a heater. The periodic supply of heat to release the fragrance composition is controlled by providing the device, and particularly the heater, with a controller. The controller is in the form of an electronic circuit. The controller is arranged such that the heater runs for a short period of time, preferably from 15 seconds to 15 minutes with "appropriate intervals of time there between."

The Whitby et al. patent application, however, appears to be directed primarily to maintaining or sustaining the olfactory impact of the fragrance composition being emitted continuously, rather than to providing to the user noticeable changes in fragrances. In addition, although the Whitby et al. patent application mentions periodic supply of two or more fragrances, there is no specific teaching of emission patterns or programs dictating the supply of the different fragrances relative to each other so that the users actually experience distinct fragrances rather than one fragrance, which is a blend of the two compositions.

### SUMMARY OF THE INVENTION

Features and Advantages of the Invention

The present inventors have discovered that the decrease in a user's scent perception is due to not only the well documented phenomenon of habituation, but also to physical, mechanical, and/or chemical changes that occur within the scent-emitting device during use. In particular, it has been found that during use, emissions from wicks diminish as a function of time, at least in part due to wick clogging. Wick clogging reduces volatilization (or evaporation), and hence the perception, of the perfume components. The clogging phenomenon can be caused by, for example, chemical reactions in the perfume composition, and gradual but selective evaporation of non-clogging perfume components. The present invention addresses these problems.

In some embodiments, the present invention provides a scent experience to a user that is more noticeable and constant over time, as compared to existing products. In some embodiments, the present invention reduces the adaptation/habituation effect by alternating perfumes. In other embodiments, the present invention provides a more efficient scent release profile of one or more perfumes. In yet other embodiments, the present invention reduces the adaptation/habituation effect and provides a more efficient perfume release profile. The present invention provides a longer lasting noticeable scent experience to a user.

Summary of the Invention

The present invention relates to a method and a device for emitting volatile compositions that contain scent compositions, insecticides, malodor control compositions, and the like. In some embodiments, the invention relates to a method for emitting one or more volatile compositions. In some embodiments, the invention relates to a method and a device for emitting two or more volatile compositions. There are numerous embodiments of the methods and devices described herein, all of which are intended to be non-limiting examples.

In some embodiments of the method, it may be desirable for those who either experience the emission of a perfume composition(s), or who are in the presence of the device(s) emitting perfume composition(s), to experience and/or perceive a pleasant scent all of the time. In other cases, this may not be all of the time, but all of the time that such persons wish to perceive a scent. In some embodiments in which the method is used to emit two or more volatile perfume compositions, it may be desirable to maximize the perceptibility of each of two or more separate and distinct volatile perfume compositions. Thus, the method can do more than merely prevent habituation to a given emitted scent. In such embodiments, therefore, it may be desirable for the time for emission of the two or more volatile perfume compositions not to change too quickly; otherwise, there will not be a perception of the different scents, but rather a blended scent. In other embodiments, however, it may also be desirable to provide a blended scent experience, for at least a period of time.

In one embodiment of the method, the volatile compositions are alternately emitted during discrete emission periods that are greater than 15 minutes and less than or equal to 24 hours. The device can automatically switch to alternate the volatile composition being emitted. In other embodiments, the device may emit volatile compositions for periods equal to 15 minutes; or it may emit volatile compositions for periods greater than 24 hours (c.g., 48 hours). Numerous other embodiments are possible.

The method can utilize one or more emission devices. In one embodiment that emits volatile perfume composition(s), a single device is used that is a dual scented electric diffuser that switches back and forth, or toggles, between two (or more) scents. In another embodiment that emits multiple volatile composition(s) such as perfume compositions and malodor control compositions, a single device is used that toggles between the compositions. In such embodiments, the emission device has a housing that is supported on an electrical outlet by a plug at least indirectly joined to the housing. The device contains a first volatile composition and a second volatile composition. The first volatile composition is emitted in an alternating period relative to said second volatile composition. Numerous other types of devices are possible. For example, in other embodiments, the method described herein can be carried out by two or more emission devices.

The present invention concerns a method for improving the emission of volatile compositions from heated-wick devices as defined in appended claim 1 and that comprise at least one porous wick that is in fluid communication with a reservoir containing a volatile composition. In one embodiment the method provides for flattening the perfume-release profile from a heated-wick perfume composition-dispensing device of a perfume composition having one or more components, comprising: a) applying heat to warm the wick to a temperature sufficient to increase the rate of volatilization of at least one component of the volatile perfume composition; b) reducing the heat to decrease the temperature of the wick sufficient to decrease the rate of volatilization of the at least one component of the perfume composition; c) maintaining the reduced heat for a time sufficient to allow for all or a portion of the components of the perfume composition to flow back through the wick toward the reservoir, or otherwise undergo diffusion to achieve equilibrium concentration within the wick ("back-flow"); and repeating a). The heat applied to a wick that is sufficient to increase the rate of volatilization of at least one component of the perfume composition can be a temperature from greater than 21 °C to 80 °C or greater, so as to achieve a wick temperature from 31°C to 80 °C or greater, or from 40 °C to 80 °C, or from 40 °C to 60°C, or from 60 °C to 80 °C. In one embodiment, the heat applied to the wick will increase the temperature of the wick over ambient in increments of approximately 10 °C. The temperature that is sufficient to achieve a decrease the rate of volatilization of the at least one component of the perfume composition can be less than or equal to 60 °C, 40 °C, or 20 °C, or less. Preferably, the temperature of the wick is reduced by 10°C from the heated temperature, and more preferably the temperature of the wick is reduced to ambient temperature. In repeated heating steps, the heat applied to the wick can result in a wick temperature that is higher than in the previous heating step.

According to the inventive method the time sufficient to allow for back-flow of all or a portion of the components of the perfume composition can be from 15 minutes to 48 hours, or from 17 minutes to 72 minutes, or from 20 minutes to 60 minutes, or 54 minutes, or 30 minutes.

Some aspects of the inventive methods include repeating steps b) and c). In some instances, a), b), and c), are each repeated at least two, three, or four times; steps a), b), and c) may be repeated a hundred or more times.

In some embodiments of the invention, the heated-wick perfume composition-dispensing device comprises at least a first and second wick, each drawing, respectively, from at least a first and second perfume composition reservoir, and the method comprises: a1) applying heat to the first wick to increase volatilization of at least one component of the first perfume composition; b1) reducing the heat applied to the first wick to a temperature sufficient to decrease volatilization of the at least one component of the first perfume composition; c1) maintaining the reduced heat applied to the first wick for a time sufficient to allow for back-flow of all or a portion of the components of the first perfume composition; a2) applying heat to the second wick to increase volatilization of at least one component of the second perfume composition; b2) reducing the heat applied to the second wick to a temperature sufficient to decrease volatilization of the at least one component of the second perfume composition; c2) maintaining the reduced heat applied to the second wick for a time sufficient to allow for back-flow of all or a portion of the components of the second perfume composition; repeating a1); and repeating a2). In some embodiments, performance of a1) and a2) overlaps for at least 0.1 seconds to 15 minutes or more. In other embodiments, the performance of a1) and a2) does not overlap.

The invention also provides a scent-dispensing system as defined in appended claim 22 comprising: a heated-wick perfume composition-dispensing device that is adapted to receive at least one perfume module, which comprises a reservoir containing a perfume composition, and a wick in fluid , communication with the perfume composition, wherein the device further comprises means for applying heat to the wick to increase volatilization of at least one component of the perfume composition; reducing the applied heat to a temperature sufficient to decrease volatilization of the at least one component of the perfume composition; maintaining the reduced heat for a time sufficient to allow for back-flow of all or a portion of the components of the perfume composition; and applying heat to the wick to increase volatilization of at least one component of the perfume composition, and wherein the device comprises means to automatically cycle through heat application and heat reduction of each wick, wherein, the time to allow for back-flow during each cycle is at least 15 minutes.

The device, in use, automatically applies heat and automatically reduces heat In some embodiments, the scent-dispensing device comprises a manually adjustable thermostat.

In some embodiments of the inventive scent-dispensing system comprises at least two compartments, each compartment or chamber being occupied respectively by each of at least two perfume compositions, and a cap that defines at least two vent holes, each vent hole positioned to cover each of said at least two compartments, said cap comprising a movable cover, which, in use, can be alternately positioned over one or more, of each of said vent holes.

The invention also provides a method for dispensing fragrance to enhance perception of at least one perfume or other volatile composition using a scent-dispensing system comprising a cover, wherein the position of the cover is automatically moved in an alternating sequence. In some embodiments the position of the cover is automatically moved in a random alternating sequence. In some embodiments, the vent holes comprise slits or louvers or both.

The inventive perfume modules can comprise one or two reservoirs. In embodiments comprising two or more reservoirs, each reservoir comprises a different perfume composition. The different perfume compositions can emit different scents or the same scents.

The wick can be made from any suitable material. For example, the wick included in the perfume module can be made from a material chosen from cellulose fibers, metal, plastic, ceramic, graphite, and cloth. In some embodiments, the wick is made from a plastic material chosen from high-density polyethylene (HDPE), polytetrafluoroethylene (PTFE), ultra high molecular weight polyethelene (UHMW), nylon 6 (N6), polypropylene (PP), polyvinylidine fluoride (PVDF), and polyethersulfone (PES). Regardless of the material of manufacture, the wick can exhibit an average pore size of from 10 microns to 500 microns, or from 50 microns to 150 microns, or an averagepore size of 70 microns. The average pore volume of the wick is from 15 % to 85 %, or from 25 % to 50 %. Good results have been obtained with wicks having an average pore volume of 38%. The wick can also be of variable length, such as, from 1 mm to 100 mm, or from 5 mm to 75 mm, or from 10 mm to 50 mm.

The invention is also directed to methods for increasing the perception of at least one fragrance dispensed from a heated-wick perfume composition-dispensing device, comprising: providing a device comprising at least first and second wicks in fluid communication with at least first and second separate perfume composition reservoirs, wherein the device dispenses fragrance by: a1) applying heat to the first wick to achieve a wick temperature sufficient to increase volatilization of at least one component of a first perfume composition; b1) reducing the heat applied to the first wick to achieve a wick temperature sufficient to decrease volatilization of the at least one component of the first perfume composition, c1) maintaining the reduced heat applied to the first wick for a time sufficient to allow for back-flow of all or a portion of the components of the first perfume composition; a2) applying heat to the second wick to achieve a wick temperature sufficient to increase volatilization of at least one component of a second perfume composition; b2) reducing the heat applied to the second wick to achieve a wick temperature sufficient to decrease volatilization of the at least one component of the second perfume composition; c2) maintaining the reduced heat applied to the second wick for a time sufficient to allow for back-flow of all or a portion of the components; repeating a1); and repeating a2).

In some methods according to the invention, the first and second perfume compositions are the same; in other embodiments, they are different*.* Different perfume compositions can exhibit different fragrances.

The present invention is also directed to methods for dispensing fragrance to enhance perception of at least one perfume, comprising: providing a device comprising at least first and second separate perfume composition-containing reservoirs, wherein the device dispenses fragrance by providing alternating bursts of emission of each of the at least first and second perfume compositions; and wherein the amount of perfume emitted per burst does not substantially vary.

In methods of the invention, the device can comprise at least first and second heaters and at least first and second wicks having a top end and a bottom end, each of the wicks in fluid communication at its bottom end with, respectively, each of the at least first and second separate perfume composition-containing reservoirs, and each of said wicks is in contact at its top end with, respectively, each the at least first and second heater, wherein the device dispenses fragrance by a1) applying heat to the first wick to achieve a wick temperature sufficient to increase volatilization of at least one component of a first perfume composition; b1) reducing the heat applied to the first wick to achieve a wick temperature sufficient to decrease volatilization of the at least one component of the first perfume composition; c1) maintaining the reduced heat applied to the first wick for a time sufficient to allow for back-flow of all or a portion of the components of the first perfume composition; a2) applying heat to the second wick to achieve a wick temperature sufficient to increase volatilization of at least one component of a second perfume composition; b2) reducing the heat applied to the second wick to achieve a wick temperature sufficient to decrease volatilization of the at least one component of the second perfume composition; c2) maintaining the reduced heat applied to the second wick for a time sufficient to allow for back-flow of all or a portion of the components of the second perfume composition; repeating a1); and repeating a2). The device is designed to automatically cycle through heat application and heat reduction of each wick, wherein the time to allow for back-flow during each cycle is at least 15 minutes, and preferably 30 minutes, and more preferably 45 minutes.

The present invention is also directed to methods for reducing the decline in the rate, over a period of time, of perfume emission from a heated-wick perfume composition-dispensing device, comprising increasing the heat applied to the wick of the device, over the period of time, wherein the increased heat is sufficient to reduce the decline in the rate of perfume emission during the period of time.

The present invention is also directed to methods for achieving an approximately constant rate of emission of perfume, over a period of time, from a heated-wick perfume composition-dispensing device, comprising increasing the heat applied to the wick of the device, over the period of time, wherein the increased heat is sufficient to achieve a wick temperature to volatilize one or more components of the perfume composition which were not volatilized at the lower heat.

Still further, the invention provides scent-dispensing systems comprising: a heated-wick perfume composition-dispensing device that is adapted to receive at least one perfume module comprising a perfume reservoir containing a perfume composition, and a wick in fluid communication with the perfume composition, wherein the scent-dispensing device, in use, automatically cycles through application and withdrawal of heat to the wick; and wherein the scent-dispensing device automatically increases the heat applied to the wick at least one time after a set time interval. The set time interval can be from 7 to 30 days, or from 7 to 15 days. The heat applied to the wick can be increased two or more times.

### BRIEF DESCRIPTION OF THE DRAWINGS

While the specification concludes with claims particularly pointing out and distinctly claiming the invention, it is believed that the present invention will be better understood from the following description taken in conjunction with the accompanying drawings in which:

Figure 1 is a diagram that shows one non-limiting embodiment of an emission program for emitting two volatile compositions.

Figure 2 is a diagram that shows one non-limiting embodiment of an emission program for emitting three (or more) volatile compositions.

Figure 3 is a diagram that shows one non-limiting embodiment of an emission program for emitting two (or more) volatile compositions where there is a gap between the emissions of the volatile compositions.

Figure 4 is a diagram that shows one non-limiting embodiment of an emission program for emitting two (or more) volatile compositions where there is an overlap of the emissions of the volatile compositions.

Figure 5 is a diagram that shows one non-limiting embodiment of an emission program for emitting three (or more) volatile compositions where there is an overlap of the emissions of one volatile composition with the emission of two other volatile compositions.

Figure 6 is a partially fragmented schematic front view showing one non-limiting embodiment of a device for emitting volatile compositions.

Figure 7 is a partially fragmented schematic side view of the device shown in Figure 6.

Figure 8 is a schematic top view of the device shown in Figure 6, showing the same adjacent to the cover plate of an electrical outlet.

Figure 9 is a perspective view of a printed circuit board that can be used to control the device shown in Figures 6-8, along with the heaters and plug that are attached thereto.

Figure 10 is a schematic of the circuit shown in Figure 9.

Figure 11 shows a cap structure which defines two vent holes and a rotatable cover comprising a grip knob.

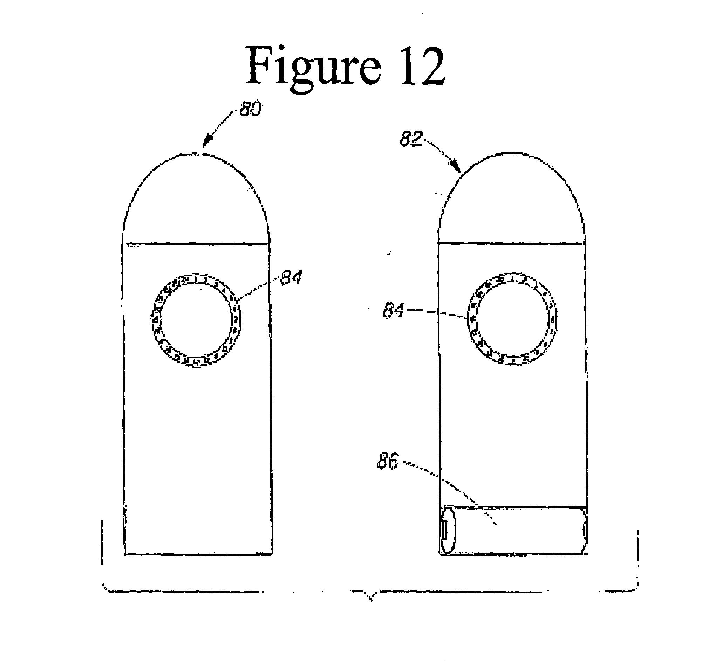

Figure 12 shows two aerosol devices that operate on timers.

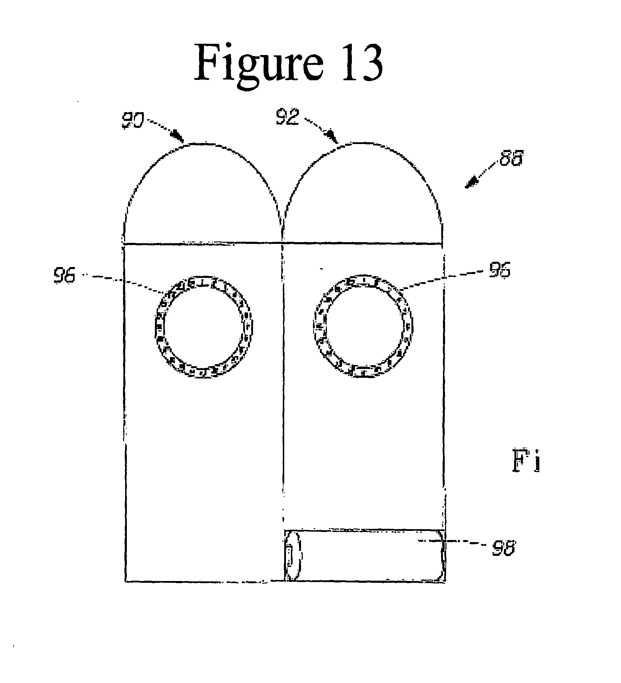

Figure 13 shows a single device comprising two aerosol containers that operate on timers.

Figure 14 diagrammatically illustrates how evaporation rate decreases over time.

Figure 15 shows photographs of wicks used continuously or toggled.

Figure 16 diagrammatically illustrates what occurs with extended continuous wick use.

Figure 17 diagrammatically illustrates how the distribution of composition components changes with time.

Figure 18 diagrammatically illustrates the effect of toggling on evaporation rates.

Figure 19 diagrammatically illustrates the effect of changing wick pore size.

Figure 20 diagrammatically illustrates the effect of changing wick length.

Figure 21 diagrammatically illustrates how changing the cycle time can improve overall scent emissions.

### DESCRIPTION OF THE EMBODIMENTS

Reference will now be made in detail to the present embodiments (exemplary embodiments) of the invention, examples of which arc illustrated in the accompanying drawings. Wherever possible, the same reference numbers will be used throughout the drawings to refer to the same or like parts.

The present invention relates to methods, devices, and systems for emitting volatile compositions. In some embodiments, the invention relates to methods and devices for emitting two or more volatile compositions. In some embodiments, the invention relates to emitting one or more volatile compositions. There are numerous embodiments of the methods and devices described herein, all of which are intended to be non-limiting examples.

The methods for emitting volatile compositions can comprise a variety of different embodiments. The volatile compositions can be fragrance compositions, compositions that function as insecticides, air fresheners, deodorants, aramacology, aromatherapy, insecticides, or any other material that acts to condition, modify, or otherwise charge the atmosphere or to modify the environment. The volatile materials emitted in a given embodiment of the method can be the same type of material (e.g., two or more fragrance compositions), or they can be different types of materials (e.g., fragrance compositions and air fresheners). Deodorants or malodor control compositions may comprise a material chosen from: odor neutralizing materials, odor blocking materials, odor masking materials, and combinations thereof. The methods can emit the volatile compositions in a sequence in which the emission of the different volatile compositions automatically alternates between the different volatile compositions.

In some embodiments, a volatile composition is emitted from a single source for a period, which is then followed by a period of decreased emission. Thus, the invention contemplates alternating, or toggling, between "on" and "off" emission of a volatile composition.

The period of emission can range from as little as 15 minutes to as long as 48 hours. Intermediate periods of emission can be 20, 25, 30, 35, 40, 45, 50, 55, and 60 minutes, and 2, 3, 4, 5, 6, 12, 18, and 24 hours, and any other intermediate time. Of course, the period of emission can range from any recited time to any recited time, for example, from 20 minutes to 24 hours, or from 30 minutes to one hour. One particular example of an emission period is 30 minutes. Another example is 45 minutes.

The period of decreased emission is similarly variable. It can range from as little as 15 minutes to as long as 48 hours. Intermediate periods of decreased emission can be 20, 25, 30, 35, 40, 45, 50, 55, and 60 minutes, and 2, 3, 4, 5, 6, 12, 18, and 24 hours, and any other intermediate time. Of course, the period of decreased emission can range from any recited time to any recited time, for example, from 20 minutes to 24 hours, or from 30 minutes to one hour. One particular example of a decreased emission period is 30 minutes. Another example is 45 minutes.

Decreased emission can be characterized by any decrease in emission. The decrease in emission can be measured quantitatively, e.g., by a decrease in the weight (mg) of a volatile composition delivered into a surrounding environment per unit time, or it can be measured qualitatively, e.g., by user perception. The decrease can be small or large, and can result in minimal or no emission. That is, emission can be reduced to its ambient level, i.e., the level of emission that occurs in the absence of deliberately applied external energy (e.g., electricity in the form of heat) added to the system; a common ambient temperature for an internal environment is in the range of from 65° F to 75 °F (18° C to 24 °C).

The present invention contemplates that two or more volatile compositions can be emitted. The two or more compositions can be the same, or they can be different. Different compositions may be designed to exhibit the same or different properties, such as the same fragrance or a different fragrance, or a fragrance and a malodor control.

Emission of the two or more volatile compositions can be performed such that the two or more compositions are emitted at the same or different times. Thus, in some embodiments, emission of the two or more compositions can entirely overlap. On the other hand, emission can be designed such that there is no overlap at all. And emission can also be designed such that there is from very small overlap in emission time to very large overlap in emission time. For example, overlap in emission time can be as little as 0.1 seconds to as much as 48 hours. Emission can be designed so that when one composition is emitted, a second is not being emitted; and when a second composition is being emitted, a first is not being emitted. Thus, the invention contemplates alternating, or toggled, emission of two or more compositions.

As noted above, emission of a volatile composition can occur for a period of as little as, for example, 15 minutes, to as long as, for example, 48 hours. The emission can be any time in between, and in some embodiments, volatile composition emission is 30 minutes. In other embodiments, volatile composition emission is 45 minutes. Where two or more compositions are emitted, a first composition can be delivered for 30 minutes ("ON"), during which time a second composition is not delivered ("OFF"). During the following 30 minutes, the first composition is OFF and the second is ON. Of course, there can be ramping toward ON and/or OFF, such that during the ramping phase, there is some overlap in emission of the compositions.

Of course, two or more volatile compositions can be emitted in any suitable sequence. The sequence of emission of the volatile compositions can be in a pattern, or it can be random. The term "pattern," as used herein, refers to repeating sequences. In embodiments where the sequence of emission of the different volatile compositions is repeatable, the pattern can be repeated once, or any number of times after the initial sequence. The term "random," as used herein, refers to sequences in which the sequence of emission of the volatile compositions does not repeat in a regular fashion. It is also possible for an emission sequence to comprise a portion of time where the sequence is in a pattern, and a portion of time in which the sequence is random.

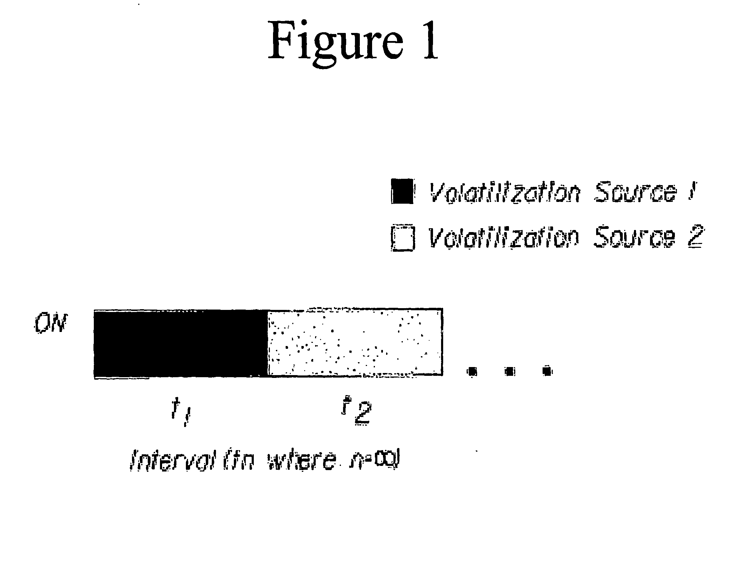

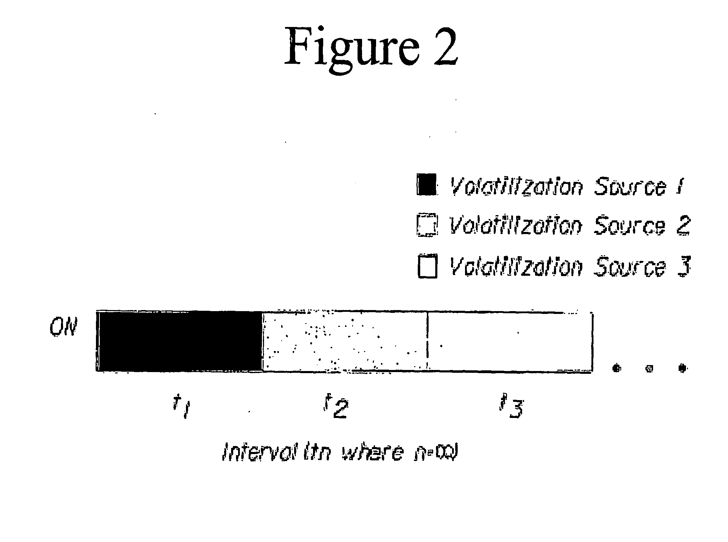

In some embodiments, two or more volatile compositions are emitted in an alternating sequence. For example, there can be a first volatile composition and a second volatile composition, and the first volatile composition is emitted in an alternating period relative to said second volatile composition. Thus, if the first volatile composition is designated "1," and the second volatile composition is designated "2," the volatile compositions can be emitted in an alternating pattern as follows: 1, 2, 1, 2, ..., etc. Figure 1 shows such an emission program schematically. In Figure 1, the diagram represents the periods during which the volatile compositions are being subjected to a source of energy (or "activated") (for example, if they are in a device which has a heater that heats the compositions, the diagram can designate the periods of time that during which the heaters are on and off). If there are three volatile compositions, they can be emitted in an alternating pattern as follows: 1, 2, 3, 1, 2, 3, ..., etc. as shown in Figure 2.

In viewing these Figures (and the diagrams which follow), it should be understood that these are non-limiting embodiments. In other embodiments, there need not be a separate volatilization source (such as a heater) for each volatile composition. There can be any suitable number of volatilization sources for the volatile compositions. For example, a single volatilization source can be used to volatilize more than one volatile composition. Such a volatilization source could, for example, be capable of moving to volatilize the different volatile compositions, or it can be capable of selectively directing energy (e.g., heat) to the different volatile compositions (such as by opening and closing a door or gate between the volatilization source and a given volatile composition). Alternatively, reservoirs can be movable relative to the volatilization source (so that the reservoirs can be selectively moved over a heater, for example).

The term "interval," as used herein, refers to the shortest period of time in the emission sequence. The term "discrete emission period," as used herein, refers to the individual time period that a given volatile material (or combination of volatile materials) is emitted in the emission sequence. This may correspond generally to the period of time that a heater, for example, is turned on for a given volatile material or combination of volatile materials (though there may be a slight lag between the operation of a heater and the emission of a volatile material). The discrete emission periods can also be referred to herein as a first time period, a second time period, etc. (each of which has a beginning and end). The term "burst" as used herein refers to an initial and peak release of a volatile composition after a heated wick is maintained at a reduced temperature to permit back flow of at least one of the components of the volatile composition. It should be understood that it is not necessary that the different volatile compositions be emitted for equal time periods. For example, after one volatile composition is emitted, a different volatile composition can be emitted for a shorter, or alternatively, a longer time period. In another example, after one volatile composition is emitted, it can be followed by another interval of the same volatile composition before a different volatile composition is emitted. In cases where the different volatile compositions are not emitted for equal time periods, it may be desirable to provide a greater amount of the compositions that are emitted for a longer cumulative time period so that the volatile compositions will be depleted at about the same time. There are numerous possible alternating emission sequences. In the case of three volatile compositions, non-limiting examples of some other possible patterns of emission include, but are not limited to: (1, 22, 1, 33); (1, 2, 3, 3, 2, 1); and (1, 2222, 11, 3333, 1).

In some embodiments of the method, the volatile compositions can be emitted during a discrete emission period that is less than or equal to 15 minutes, but it may be more desirable for each emission period to be longer than 15 minutes. In the case of scented materials, longer time periods may be more desirable. In one embodiment of the method, the volatile compositions are alternately emitted during discrete periods that are each greater than 15 minutes and less than or equal to 12 hours, or less than or equal to 24 hours, or less than or equal to 48 hours, or more. Every numerical range given throughout this specification will include every narrower numerical range that falls within such broader numerical range, as if such narrower numerical ranges were all expressly written herein. Thus, in another non-limiting embodiment, the volatile compositions are alternately emitted during periods that are greater than 15 minutes, or greater than or equal to 1 hour, and less than 2 hours. In one embodiment, each volatile composition isemitted for aperiod of 72 minutes. In one embodiment, each volatile composition is emitted for 30 minutes.

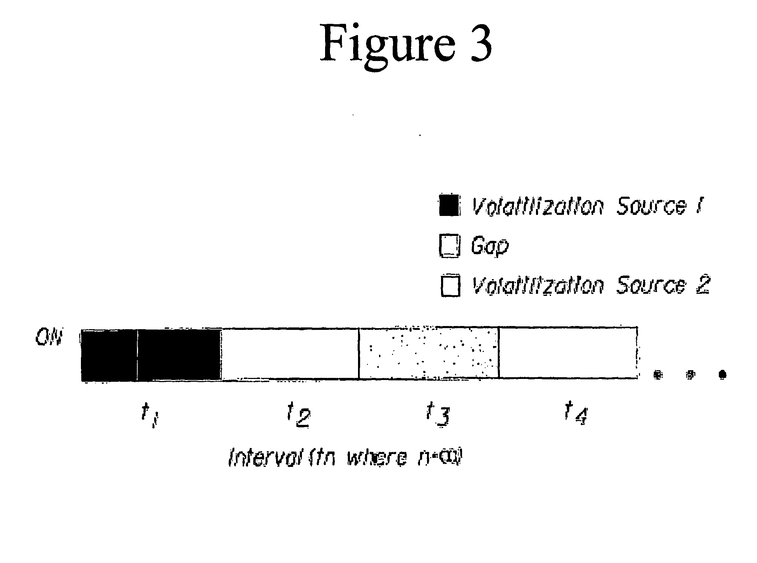

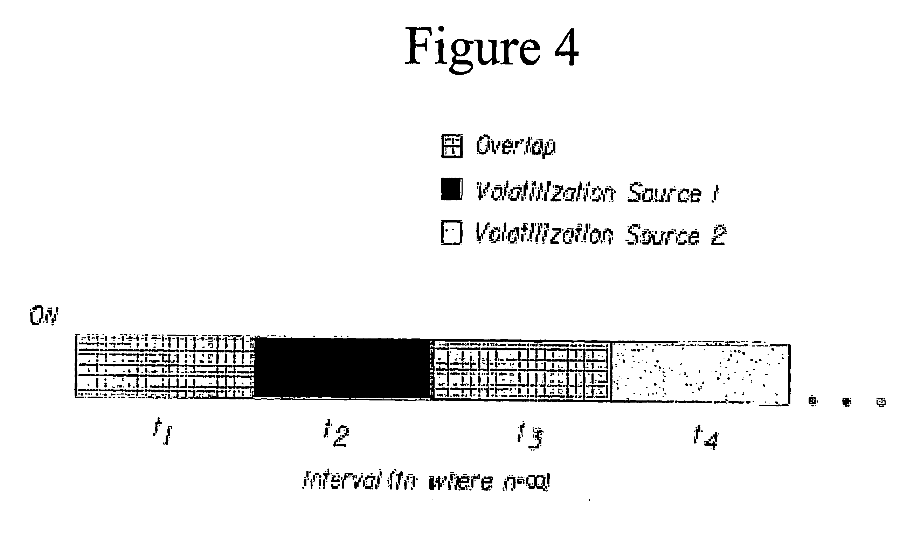

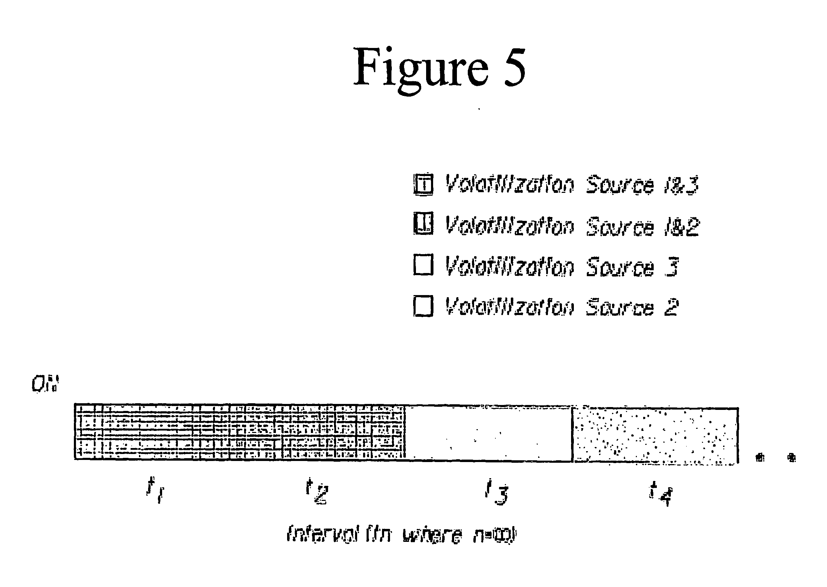

The volatile compositions may be emitted so that one immediately follows the end of the emission period of the other. In other embodiments, the volatile compositions can be emitted so that there is a gap between the end of the emission period of one of the volatile compositions, and the beginning of the emission period of another volatile composition. Figure 3 is a diagram that shows one non-limiting embodiment of an emission program for emitting two volatile compositions where there is a gap between the emissions of the volatile compositions where "g" designates a gap. In other embodiments, the volatile compositions can be emitted so that there is an overlap in the emission periods of two, or more volatile compositions. Figure 4 is a diagram that shows one non-limiting embodiment of an emission program for emitting two volatile compositions where there is an overlap of the emissions of the volatile compositions where the symbol "&" designates an emission period where both volatile compositions are being emitted. Figure 5 shows one non-limiting embodiment of an emission program for emitting three (or more) volatile compositions where there is an overlap of the emissions of one volatile composition with the emission of two other volatile compositions. In other embodiments, it is possible for one or more of the volatile compositions to be emitted continuously, and another volatile composition to be emitted for periods of time that are greater than 15 minutes.

If it is desirable to have a gap between the end of the emission period of one of the volatile materials, and the beginning of the emission period of another volatile material, the gap can be of any suitable duration. The gap period of between emissions of volatile material may be from greater than 0% up to 100% or more of the duration of either the previous or subsequent emission periods. If it is desirable to have an overlap in the emission periods of two, or more volatile materials, the overlap can be of any suitable duration. The emission period of a subsequently emitted volatile material may overlap from greater than 0% up to 100% of the time a first volatile material is being emitted. In certain embodiments, for example, it may be desirable for there to be an overlap of about 25% between different volatile materials. For instance, instead of scent "A" being emitted for 60 minutes, followed by scent "B" being emitted for 60 minutes: scent "A" can be emitted for 45 minutes; this can be followed by the emission of both scents "A" and "B" for 30 minutes; and this followed by scent "B" for 45 minutes. In this case, 30 minutes is 25% of the total time of the emission of scents "A" and "B" and the combination thereof (or 120 minutes).

The gap or overlap periods can be controlled automatically. In certain embodiments of the article(s) or device(s) used to emit the volatile materials, the article(s) or device(s) can be provided with controls to allow the user to control the duration of any gap and/or overlap in emission periods. Overlapping sequences may be used for any purpose, such as, for example, when it is desirable to have the user smell the blended scent for some period as well as distinct scents during other periods.

In certain embodiments, it is desirable for the method to be carried out by article(s) and/or device(s) that are flameless (e.g., not candles). In certain embodiments, it may be desirable for the method to be carried out independently of other media (such other media may include, but is not limited to: movies, television, etc.). In other embodiments, it may be desirable to carry out the method in a coordinated fashion with other media.

There can be any suitable emission program or scheme for emitting the volatile compositions. In certain embodiments where scented materials are being emitted, it is desirable for the device to provide an alternating scent experience, rather than a sustained impression of a single scent. In one embodiment, it may be desirable to provide a day/night emission program where one scent is provided for waking a person, and another scent is provided for the period of time during which they are trying to go to sleep. Thus, in some embodiments, it may be desirable to deliver the same scent at the same time every day. In other embodiments, it may be desirable to avoid a routine scent experience. For example, it may be desirable for the emission pattern to not be synchronized over a 24-hour period, so that the user has a different scent experience at a given time during the day or night for each 24-hour period*.* Numerous other embodiments are possible.

The total emission program (or simply "the emission program") refers to the entire sequence of the discrete emission periods from beginning to end. In certain embodiments, it is desirable for the emission program to be continuous. The term "continuous," as used in reference to the emission program, means that there is a planned emission sequence over an entire period, once the program is initiated. This emission program can include periods, as noted above, where there are gaps in emission. This will still be considered to be a continuous emission program, although there will not necessarily be continuous emission of volatile compositions. It should be understood, however, that it is possible for the emission program to be interruptible by the user (e.g., turned off), if desired. Thus, the method can provide a user interface, and the user interface can provide a user with the ability to interrupt the emission program. In certain embodiments, the emission program may be designed to run continuously or substantially continuously until at least one of the volatile compositions is substantially depleted. In certain embodiments, it is desirable for the emission program to run continuously until all of the volatile compositions are substantially depleted, and for this to occur at approximately the same time. The emission program can be of any suitable length, including but not limited to 30 days, 60 days, or shorter or longer periods, or any period between 30 to 60 days.

One example of a device that can be used in accordance with this invention is one that includes a wick. When used in such devices, the wick acts as a conduit to carry a volatile composition from a reservoir to a point of emission. A wick is generally porous, or includes pores, that provide for the flow of the volatile composition. Wicks can be made from a variety of materials, including but not limited to, cellulose fibers, metal, plastic, ceramic, graphite, and cloth. Synthetic materials, such as plastic, may be desirable because of their uniformity in performance. Plastic materials that can be used to form porous wicks include, but are not limited to, high-density polyethylene (HDPE), polytetrafluoroethylene (PTFE), ultra-high molecular weight polyethylene (UHMW), nylon 6 (N6), polypropylene (PP), polyvinylidine fluoride (PVDF), and polyethersulfone (PES).

Wicks can be described in terms of their average pore size. The wicks may have any suitable pore size. US Patent Application 2002/0136886 A1, titled "Porous Wick for Liquid Vaporizers" provides a description of standard measurement of pore size. In certain embodiments, the average pore size of wicks useful in the present invention ranges from 10 microns to 500 microns, or from 50 microns to 150 microns, or from 60 to 100, or 70 microns. Wicks can have an average pore volume from 15 % to 85 %, or from 25 % to 50 %. Similarly, wicks can vary in length, depending solely on the desired use. In certain embodiments, wicks can be as short as 1 mm or as long as 100 mm, or longer, or any length in between. Wicks can range in length from 5 mm to 75 mm, or from 10 mm to 50 mm

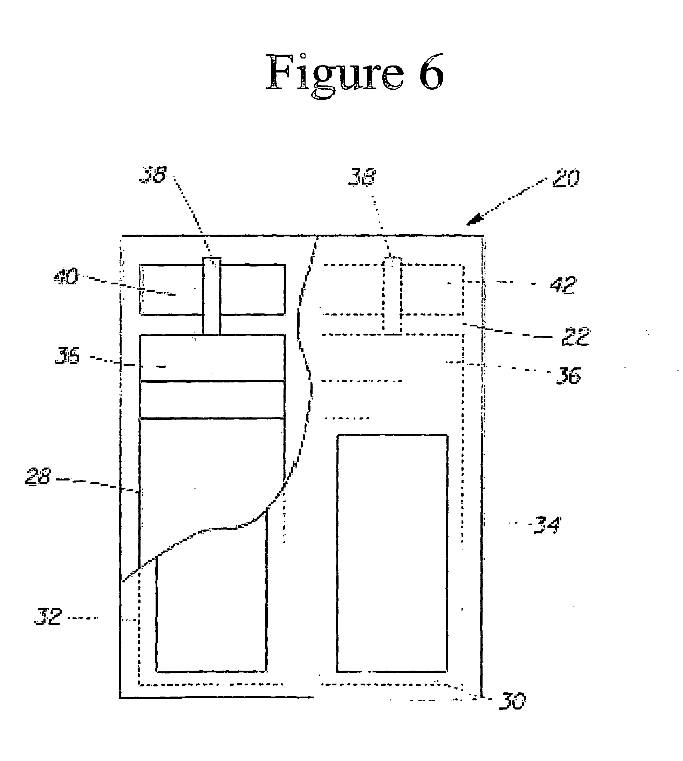

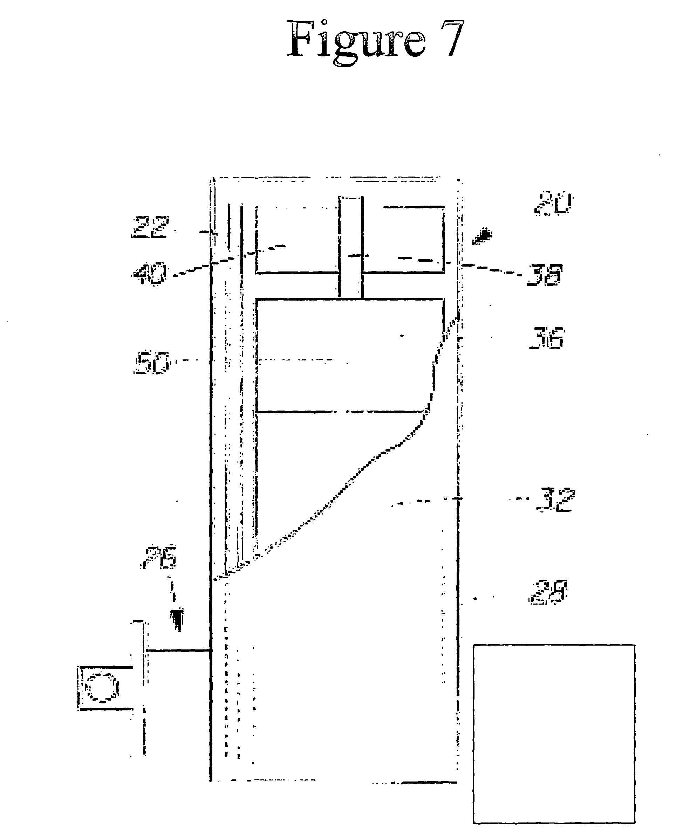

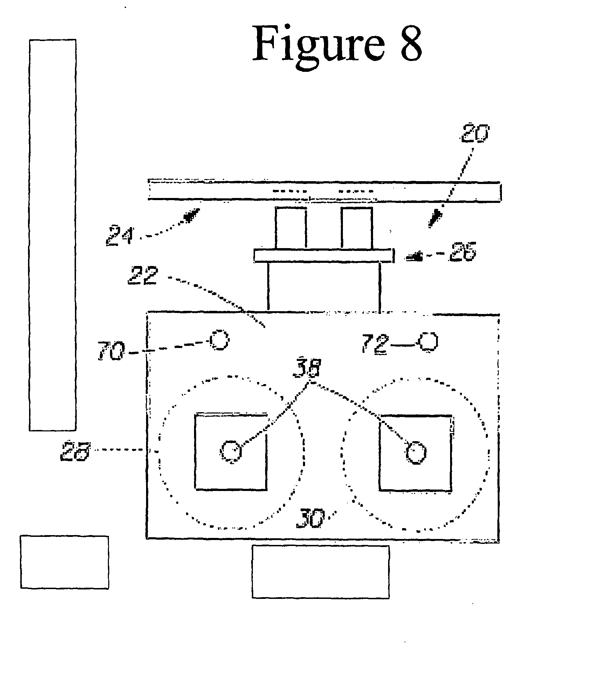

Figures 6-8 show one non-limiting embodiment of a device 20 for emitting volatile compositions according to the methods described above. The device can have a pre-selected emission program, which is already programmed when a consumer buys the device, or the device can be provided with a selection of several emission programs and the consumer can select between these programs. In these or other embodiments, the device 20 can use technology similar to the "random play" technology used in compact disc (CD) players to randomly alternate between different volatile materials.

As shown in Figures 6-8, the device 20 comprises a housing 22, and the housing 22 is supported on an electrical outlet 24 by a plug 26 that is at least indirectly joined to the housing 22. The device 20 further comprises at least one container, or reservoir. In the embodiment shown in Figures 6-8, the device 20 comprises two reservoirs 28 and 30. The reservoirs 28 and 30 contain at least a first volatile composition 32 and a second volatile composition 34. The housing 22 may serve as a holder for the reservoirs 28 and 30 and any of the other components of the device described below.

The reservoirs 28 and 30 can comprise any suitable type of container, and can be made of any suitable material. Suitable materials for the reservoirs include, but are not limited to glass and plastic. The reservoirs 28 and 30 can comprise any type of container that is suitable for holding volatile materials. The reservoirs 28 and 30 may be part of the housing 22, or they may be separate components that are removably joined to a portion of the device 20 such as the housing 22. It is also possible for a single reservoir to hold more than one type of volatile material. Such a reservoir could, for instance, have two or more compartments for volatile materials. In the embodiment shown in Figures 6-8, the reservoirs 28 and 30 comprise two separate bottles.

The reservoirs 28 and 30 in Figures 6-8 contain volatile compositions in the form of scented perfume oils. The reservoirs further comprise a seal 36 for containing the volatile material, and a wick 38 for dispensing the volatile material. The device 20 and/or the reservoirs 28 and 30 may further comprise an additional seal for covering the wick 38 of one or more of the volatile materials when the volatile material is not being emitted.

The term "volatile compositions" as used herein, refers to a material or a discrete unit comprising of one or more materials that is vaporizable, or comprises a material that is vaporizable. The term "volatile compositions," thus includes (but is not limited to) compositions that are comprised entirely of a single volatile material. The terms "volatile materials," "aroma," "fragrance," and "scents," as used herein, include, but are not limited to pleasant or savory smells, and, thus, also encompass materials that function as insecticides, air fresheners, deodorants, aromacology, aromatherapy, insecticides, or any other material that acts to condition, modify, or otherwise charge the atmosphere or to modify the environment. It should be understood that certain volatile compositions including, but not limited to perfumes, aromatic materials, and scented materials, will often comprise one or more volatile materials (which may form a unique and/or discrete unit comprised of a collection of volatile materials). It should be understood that the term "volatile composition" refers to compositions that have at least one volatile component, and it is not necessary for all of the component materials of the volatile composition to be volatile. The volatile compositions described herein may, thus, also have non-volatile components. It should also be understood that when the volatile compositions are described herein as being "emitted," this refers to the volatilization of the volatile components thereof, and does not require that the non-volatile components thereof be emitted. The volatile compositions of interest herein can be in any suitable form including, but not limited to, solids, liquids, gels, encapsulates, wicks, and carrier materials, such as porous materials impregnated with or containing the volatile material, and combinations thereof.

In the case of scented materials or fragrances, the different scented materials can be similar, related, complementary, or contrasting. It may not be desirable, however, for the scented materials to be too similar if the different scented materials are being used in an attempt to avoid the problem of scent habituation; otherwise, the people experiencing the scents may not notice that a different scent is being emitted. The different scents can be related to each other by a common theme, or in some other manner. For example, the different scents can all be floral, fruit scents, etc. An example of scents that are different, but complementary, might be a vanilla scent and a French vanilla scent.

The wick devices are heated wick devices, as further described herein.

The embodiment of the device 20 shown in Figures 6-8 further comprises a mechanism for activating the volatile materials from their "resting" state to an activated state. Such a component may include, but is not limited to, a component that volatilizes or heats the volatile materials. The device 20 may also contain a component, such as a fan, for diffusing or transporting the volatile materials into the environment or atmosphere*.* In various embodiments, the device 20 may comprise a heater, a fan, or both, or some other type of mechanism.

In the embodiment shown in Figures 6-8, the device 20 comprises at least one heating system or heater, such as heaters 40 and 42. The heaters 40 and 42 can comprise any suitable type of heater, and can be located in any suitable location in or relative to the device 20. In the embodiment shown in Figures 6-8, the heaters 40 and 42 comprise heating elements that are in the form of circular rings that at least partially surround the wicks 38 protruding from the bottles of the volatile compositions.

' The device 20 shown in Figures 6-8 further comprises a switching mechanism 50 that changes the volatile material being emitted by the device 20. The switching mechanism 50 can comprise any suitable type of mechanism that causes the device to change the volatile material being emitted. In the embodiment shown, the switching mechanism controls the activation of the heaters so that the heater will be turned on for the volatile material that is desired to be emitted. Suitable switching mechanisms include, but are not limited to, analog timing circuitry, digital circuitry, combinations of analog and digital circuitry, microprocessors, and mechanical actuation switches such as shape memory alloys (NiTi wire) or bimetallic switches.

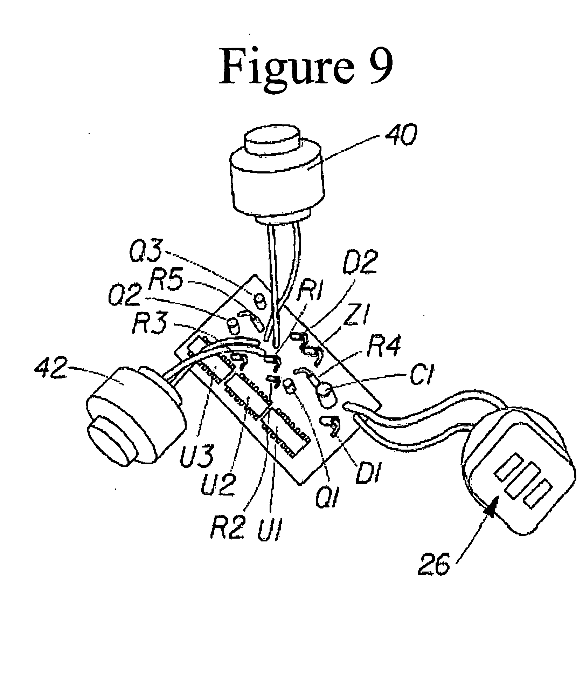

As shown in Figure 9, in one non-limiting embodiment, the switching mechanism 50 comprises a combination analog and digital circuit in the form of a printed circuit board (or "PCB"). The circuit comprises: a single-sided PC board 52; a capacitor designated C1; a pair of diodes D1 and D2; three transistors Q1, Q2, and Q3; five resistors R1-R5; three counters U1, U2, and U3; a third diode Zl. Any suitable type of heater can be used for heaters 40 and 42, including but not limited to resistance heaters (several types of which are commercially available). The heaters 40 and 42, as well as the wall power plug 26, are also connected to the circuit board 52 by wires 66. Suitable components for circuit are set out in the following table:

**Table 10**

| Reference Number or Letter | Component | Properties |
|---|---|---|
| C1 | Capacitor, Electrolytic | 1 microF, 250 V |
| D1, D2 | Diode | 1N4004, or similar |
| 26 | Wall power plug | |
| Q1, Q2, Q3 | Transistors. NPN | NPN 200V, 200mA |
| R1-R5 | Resistors | 1/8 watt |
| U1, U2, U3 | Counters | CD4024, or similar |
| Z1 | Diode, Zener, 11V | 1N4741A, or similar |

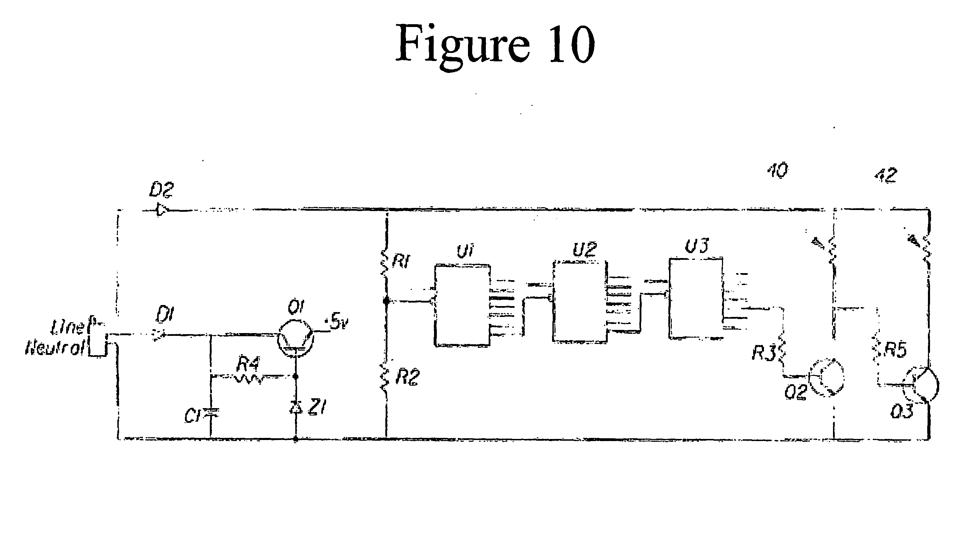

The components of the circuit may be through-hole or surface mounted. In the embodiment shown, a 38 X 66 mm single sided PC board 52 with through-hole components is used. The material comprising the PC board 52 can be a standard material such as FR-4 epoxy base fiberglass, but any UL approved material is acceptable. The wall power plug 26 is a molded wall plug with approximately 100 mm pigtails into the PC board. Figure 10 is a schematic for one example of a circuit. This circuit provides a timing function that alternates current between two paths over a time period of several tens of hours, with a pre-selected time for each heater to be turned on and off.

In other embodiments, the switching mechanism may include, but is not limited to, the following alternative types of switching mechanisms: (1) a magnetic sensor with a pickup that counts the number of rotations of the motor of a fan used to disperse the volatile composition(s) such that after a certain number of rotations, the device will switch from one volatile composition to another; and (2) a device comprising dual shape memory alloys, or bimetallic strips or switches that can complete a circuit at ambient temperature and then cut-off when a certain temperature is reached. The two-way effect can be used since as the temperature lowers, the material can complete the circuit again, thus acting as a thermostat to keep the heater on and then turn it off. The shape memory alloy may serve as the heater as well as the pulse generator.

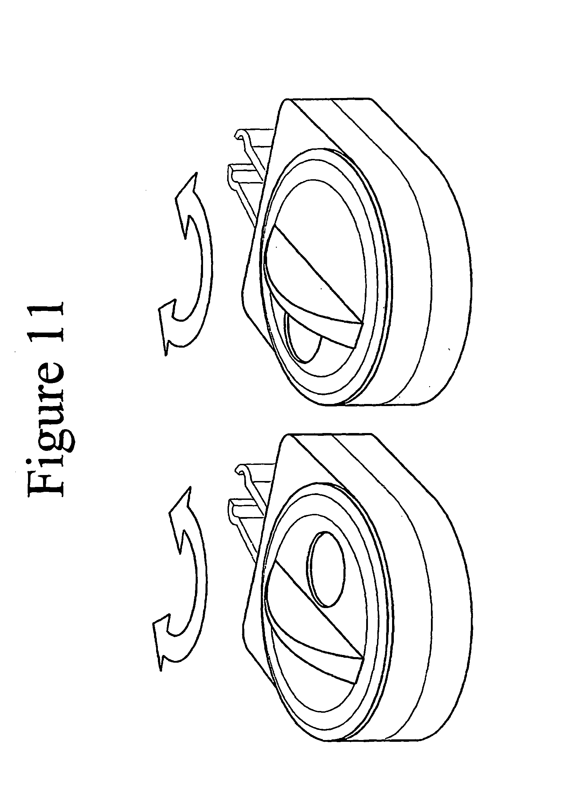

Other embodiments of switching mechanisms include movable covers to control the release of one or more volatile compositions. In certain embodiments, the devices for dispensing volatile compositions comprise a cap or other structure which encloses one or more chambers or is otherwise positioned in relation to two or more positions or spaces which are occupied by two or more separate units or modules for dispensing volatile compositions. In other embodiments, the devices for dispensing volatile compositions comprise caps or other structures which cover two or more separate units or modules for dispensing volatile compositions. The cap structures define two or more vent holes or orifices, and optionally comprise vents, louvers or combinations thereof*.* The cap structures comprise a movable cover which can be automatically or manually moved to cover one or more of the discrete chambers or spaces, to enclose or reveal the two or more separate units or modules for dispensing volatile compositions. In one embodiment, the cover, in use, is moved to alternately cover at least one chamber while exposing the space defined by at least one chamber. Optionally, the cap may have a clip structure which facilitates fixation or positioning of the volatile composition-dispensing device on an architectural structure of a house, or on a piece of furniture or an automobile fixture. Figure 11 depicts a cap structure which defines two vent holes and a rotatable cover which is manually actuated by the rotation of a knob to selectively expose one or the other, or neither of the vent holes. In some embodiments, the cap structure may be automatically operated. In some embodiments, the device may further comprise a fan in operable communication with the cap structure to facilitate the volatilization of the volatile compositions from within the vented portion or portions of the device or system. Particularly useful volatile composition-dispensing modules and materials for use with devices having cap structures include passive, or unheated, wicks, liquids, slurries, gels, and solid beads.

The device 20 can comprise a number of additional optional features. The device can be provided with indicators so that a person is further made aware that the volatile material being emitted has changed. Such indicators can be visual and/or audible. For example, in the case of scented materials, such an indicator may allow a person to see which scent is being emitted at a given time. In the embodiment shown in Figures 6-8, the indicators are in the form of lights 70 and 72. In another example, at least a portion of the device 20 (such as all or a portion of the housing) or the reservoirs may be made of a type of plastic that changes color when heated.

The device can be provided with additional user controls. The device can include an "on/off" switch to allow a user to turn the device on and off without removing it from the electrical socket. The device can be provided with a control that allows the user to control the emission period of one or more of the volatile compositions, and/or the time between the emission of the different volatile compositions, or the time that the volatile materials are emitted during an overlapping time period. For example, in one non-limiting embodiment, if the device is provided with the capability of emitting each volatile material during a period greater than 15 minutes and less than or equal to 48 hours, then the device can be provided with a control that allows the user to set the emission period for one or more of the volatile compositions to 30 minutes, 45 minutes, or 72 minutes, or to one hour, for example.

The device can be provided with additional user controls. The device can comprise a thermostat or other switch to allow a user to adjust the temperature settings of the heat sources for one or more of the volatile compositions. The settings may be predefined for particular volatile compositions, or may be adjustable based on selected temperatures to be applied to a wick.

The device can be battery powered so that it need not be plugged into an electrical outlet. The device can also be configured so that it can be both plugged in and powered by a source of electrical current, and also battery powered. The device can also be provided with an adapter so that it can be plugged into the cigarette lighter in a vehicle. In addition, the device can be provided with a remote control that allows the user to control any, or all, of the emission properties of the device (including, but not limited to changing the volatile material being emitted) without touching the device.

The device may comprise a microprocessor that has less component parts compared to analog circuits, and improved circuit quality from lot to lot. The microprocessor can allow the user to program and control the temperature profile by modulation to alter performance. If desired, the microprocessor may be connected to a user interface, This can be any suitable type of user interface. Examples of types of user interfaces include, but are not limited to LCD screens and LEDs. In addition, the microprocessor enables components to allow multiple devices (such as those located in different parts of a room, or in different rooms), to communicate with each other. For example, the microprocessor can enable a remote control to send digital signals via an infrared beam to turn another device "on" or "off."

In some embodiments, the devices can be configured to turn on and off in response to some stimulus, such as by sensors that respond to light, noise and/or motion. For example, one of the devices can be configured to turn on when it senses light, and another device can be set to turn off when it senses light. In another example, a microprocessor can be used with motion sensors to turn on the device (for example, a heater, and/or a fan in the device). For example, the device can be off all the time until a person moves in the vicinity of the motion sensor. The device can then turn on when a person walks in the vicinity of the motion sensor. Using a microprocessor provides flexibility in controlling the characteristics of the emission of the volatile materials. This is because it is possible to replace the microprocessor if it is desired to change the emission characteristics. Replacing the microprocessor eliminates the need to modify the entire circuit.

### Examples

Examples 4-7 are provided for comparative purposes.

Example 1: Perfume Evaporation in Plug-Ins Decreases Overs Time

It is known in the art that extended exposure to a scent produces an habituation effect, whereby a person is less able to recognize the presence of a particular scent, even if present in the same concentration. This phenomenon occurs with the use of scent-emitting devices that are commercially available.

However, it is believed by the present inventors that, in addition to the habituation phenomenon, reduced scent output over time by commercially available devices even further contributes to lack of user scent recognition. To test this hypothesis, a commercially available product, GLADE ® Sky Breeze ®, was plugged in (i.e., turned on) and allowed to emit scent for an extended period of time. Evaporation rate (or release rate) was determined by measuring the starting content of the device and taking daily measurements to determine how much was lost.

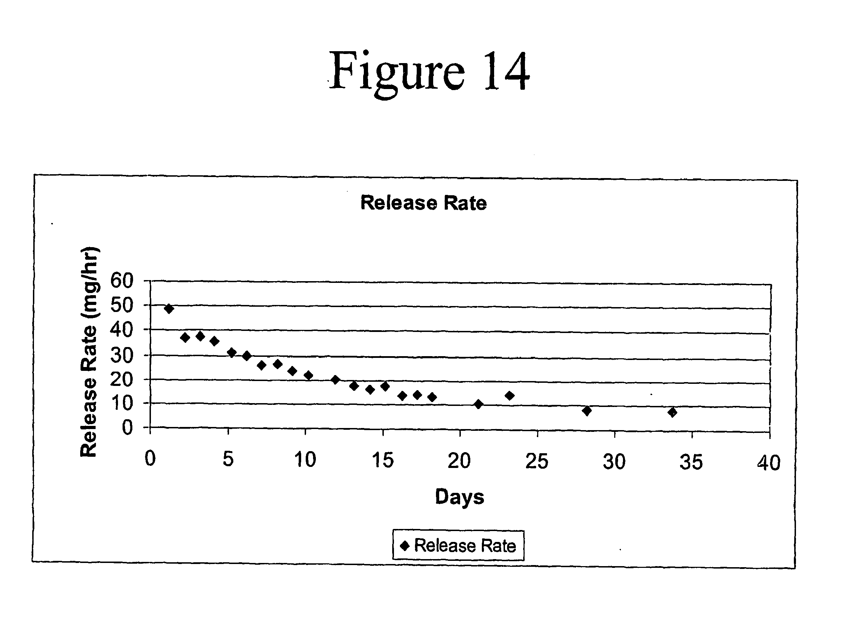

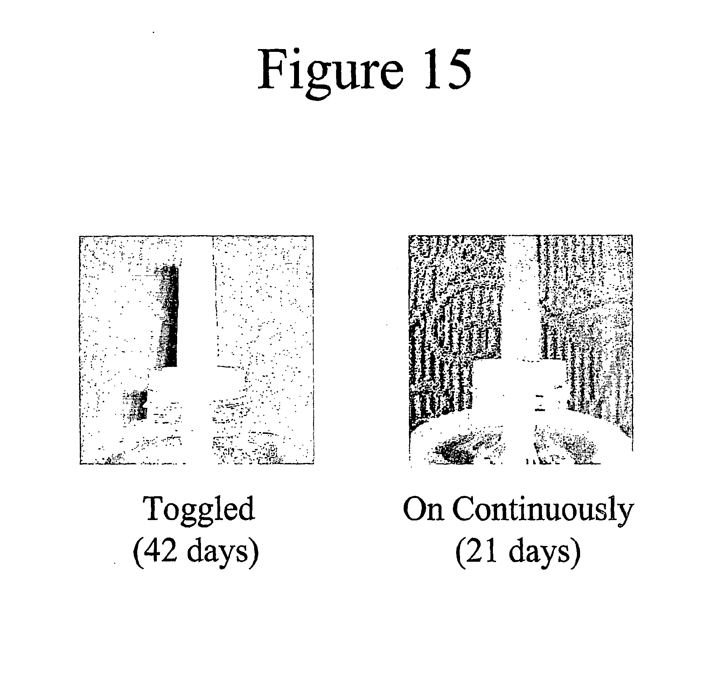

Figure 14 shows the results of the study. As can be seen from the Figure, evaporation rate significantly decreases over time. Indeed, evaporation rate had fallen by nearly 50% after only one week of use. Additionally, there is a visible difference in wicks that are used continuously versus toggled. (See Figure 15, which shows a photo of a wick that was used continuously for 21 days, compared to a photo of a wick toggled on and off for 42 days.)

Example 2: Wick Clogging Causes Decrease in Evaporation Rate

The observed reduction in evaporation rate could have been caused by a number of factors, including selective evaporation of more volatile compounds and clogging of the wick. To determine the mechanistic reasons behind the decrease in evaporation rate, further studies were performed.

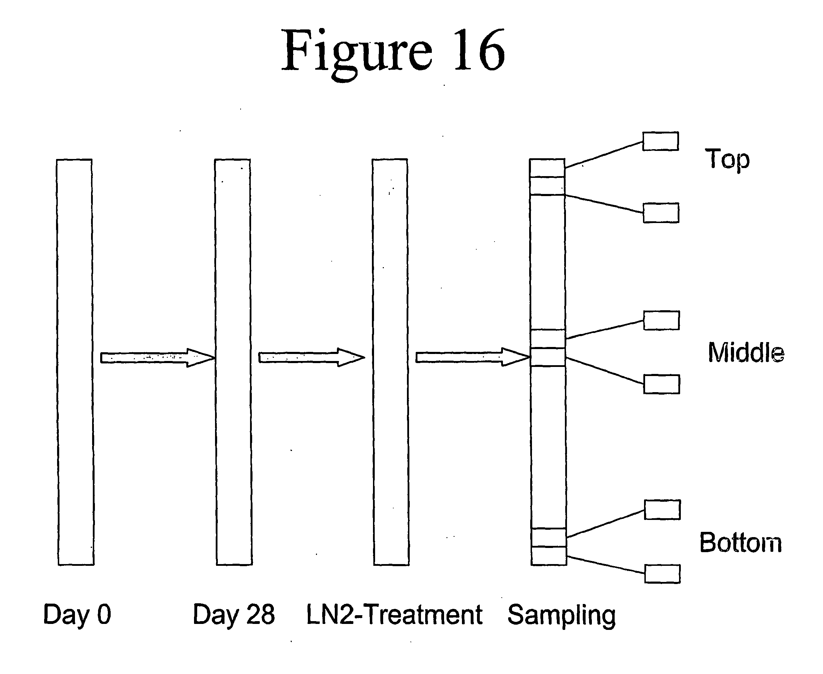

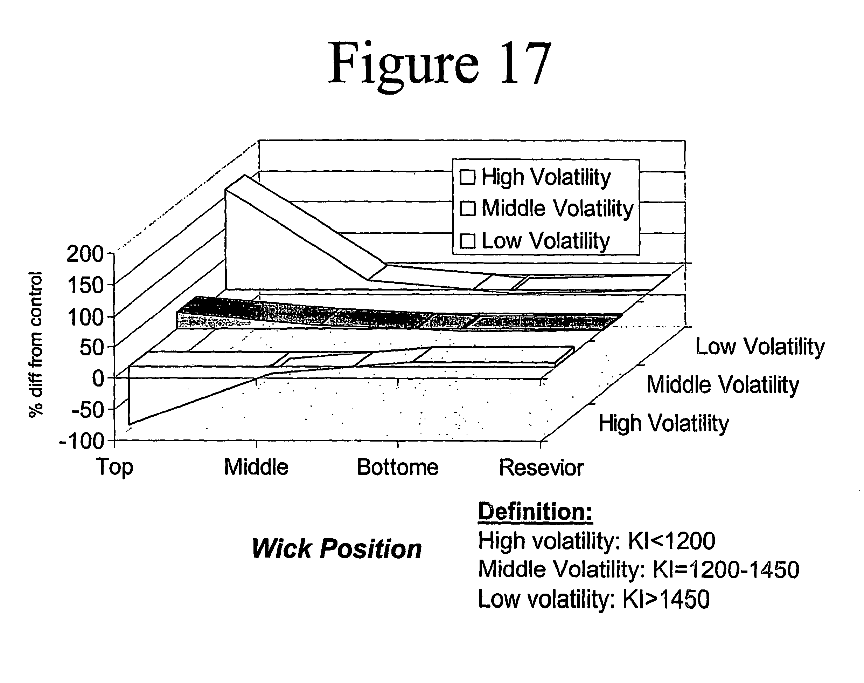

GLADE ® Vanilla Breeze ® and Hawaiian Breeze ® perfume devices were turned on for 28 days continuously. At the end of 28 days, the wick was removed from the device and frozen in liquid nitrogen. Samples were taken from the top, middle, and bottom of the wick. At the same time, the volatile composition remaining in the reservoir was sampled. The volatile compositions sampled were analyzed by gas chromatograph and their Kovats indices determined. Figures 16 and 17 diagrammatically illustrate the results.

As can be seen from Figure 16, the wick visibly changes over the testing period. The bottom of the wick is lightest in color and the wick becomes darker toward the top. This is also apparent in the photos in Figure 15.

Figure 17 shows exactly what happens during wick clogging. There was almost no difference between the different components in the content of the reservoir: low, middle, and high volatility components differed very little from control, and differed very little from each other. The same was observed for the bottom and middle of the wick.

The top of the wick, however, showed striking differences. High volatility compounds were nearly depleted and middle volatility compounds were slightly higher than control. Most striking, low volatility compounds had collected at the top of the wick more than 150% more than control. The population of volatile compounds at the top of the wick -- the primary locus of volatilization - was very highly skewed toward low volatility components. Thus, these low volatility components were effectively controlling the rate of evaporation, in effect "clogging the wick."

Without wishing to be bound by theory, it appears that what is occurring is a build-up of materials having lower volatility at the top of the wick, thereby preventing more volatile materials from moving to the top of the wick and evaporating. At the same time, compounds in the volatile composition that are more volatile are selectively evaporated, further concentrating lower volatility compounds in the top of the wick, and further aggravating the clogging phenomenon. Thus, for a number of reasons that may be additive or even synergistic, evaporation of volatile materials rapidly decreases during prolonged use of a wick device. The energy continually applied to the device, in the form of sustained heat to the wick, drives these less volatile compounds against the concentration gradient, forcing them to accumulate in the top of the wick. The emission from a commercial plug-in product was shown to drop by about 50% during one only one week's use. (See Figure 14.) By deliberately toggling a heated wick device to the "OFF" position, or introducing a gap between the emissions of a single or multiple wick device, the volatile components diffuse within the wick to reach an equilibrium concentration that approximate the composition of the volatile component mixture in the reservoir. Thus, toggling "OFF," or providing a gap in the emission by removing heat from the wick relives the concentration gradient that "clogs" the wick.

Example 3: Toggling Reduces Clogging and Improves Evaporation Rates

Once it was discovered that the reduction in evaporation rate was caused by wick clogging, steps were taken to solve the problem. It was surprisingly discovered that by allowing the wick-based evaporation device a resting period (generally by reducing the heat applied to the wick), back-flow of volatile materials occurs within the wick, thereby allowing the less volatile components to flow with the concentration gradient and back toward equilibrium. Once the resting period is over, and back-flow of the less volatile components has occurred, energy can be reapplied to the system for a finite period, during which volatilization of the composition again occurs. This cycle can be repeated any number of times.

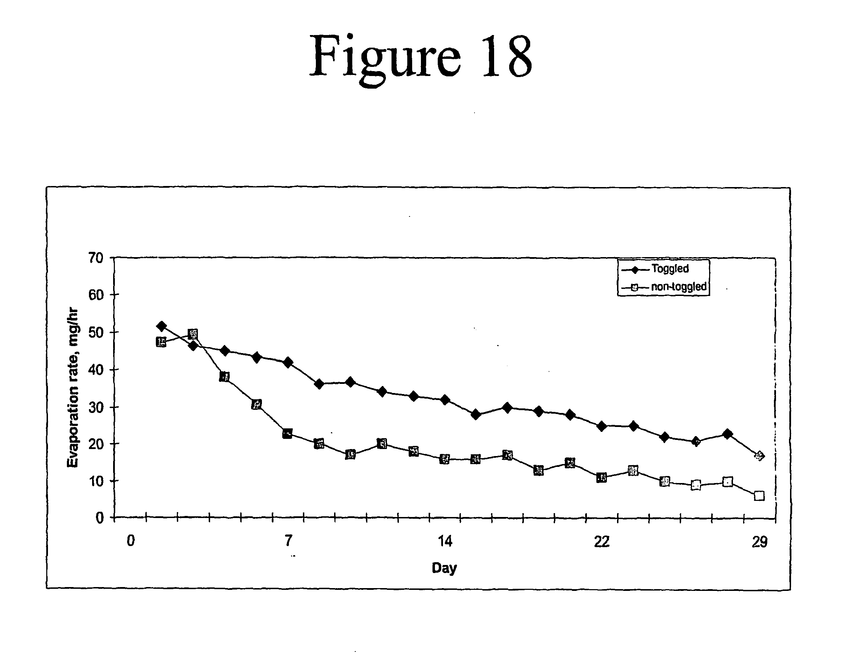

Figure 18 shows that by toggling a single wick device on and off, an evaporation rate can be improved significantly. Briefly, Figure 18 shows two evaporation curves for a commercially available product, GLADE ® Sky Breeze ®, which was activated in two different ways. One device remained on continuously for approximately four weeks ("non-toggled"). The other device was cycled on and off for 72-minute intervals ("toggled") over an eight-week period. (Testing of the toggled device required twice as long, so the "days" axis values were divided by two to achieve a comparable curve.)

As can be seen from Figure 18, cycling a wick device on and off produces a significant increase in the evaporation rate from the device. The photos in Figure 15 provide evidence that the on/off cycling significantly reduces the wick clogging.

Example 4: Larger Pore Size Improves Evaporation Profile

Once it was discovered that wick clogging was responsible for reduced evaporation, and that by reducing clogging evaporation could be increased, further steps were taken to identify ways to improve evaporation. This Example shows that by increasing pore size, evaporation is increased.

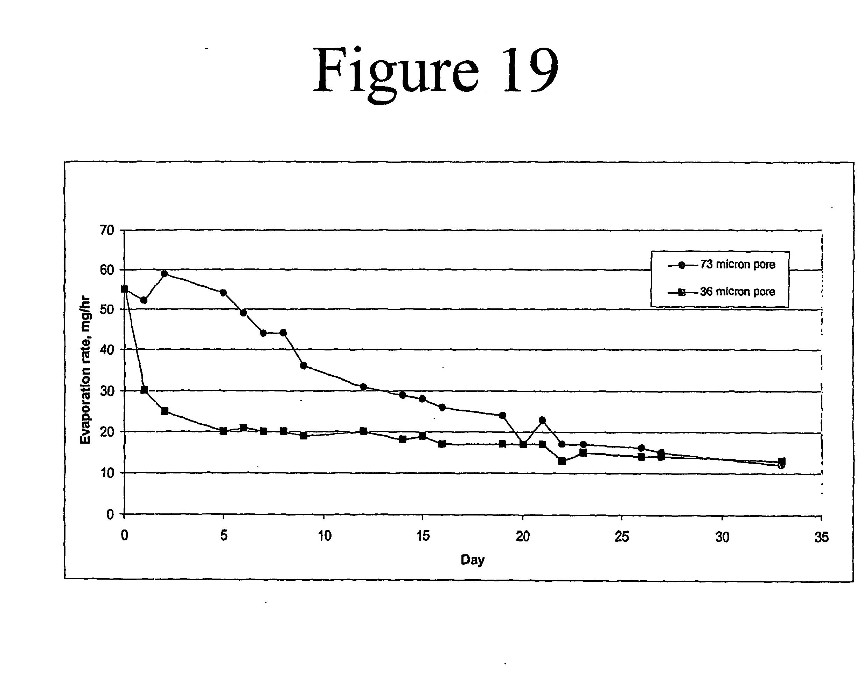

Briefly, an AIR WICK ® Country Berries ® perfume device was tested using two wicks: a) 36 micron average pore size, and b) 73 micron average pore size. The two wicks were tested by running a device continuously for more than one month by toggling on and off. Figure 19 shows the results.

As can be seen, within the first week, the higher pore size wick performed significantly better than the smaller pore-size wick. The difference decreased over the second week, and by the third week, there was no difference. It is believed that this convergence at a point of low performance results from wick clogging. The wick clogging occurs in the low pore size wick much earlier than in the larger pore size wick. From these experiments, it can be concluded that a larger pore size wick produces better evaporation than a smaller pore size wick.

Example 5: Shorter Wicks Perform Better

Additional studies were performed to determine if other characteristics of the wick could improve the evaporation profile. This example shows how by decreasing wick length, evaporation can be improved.

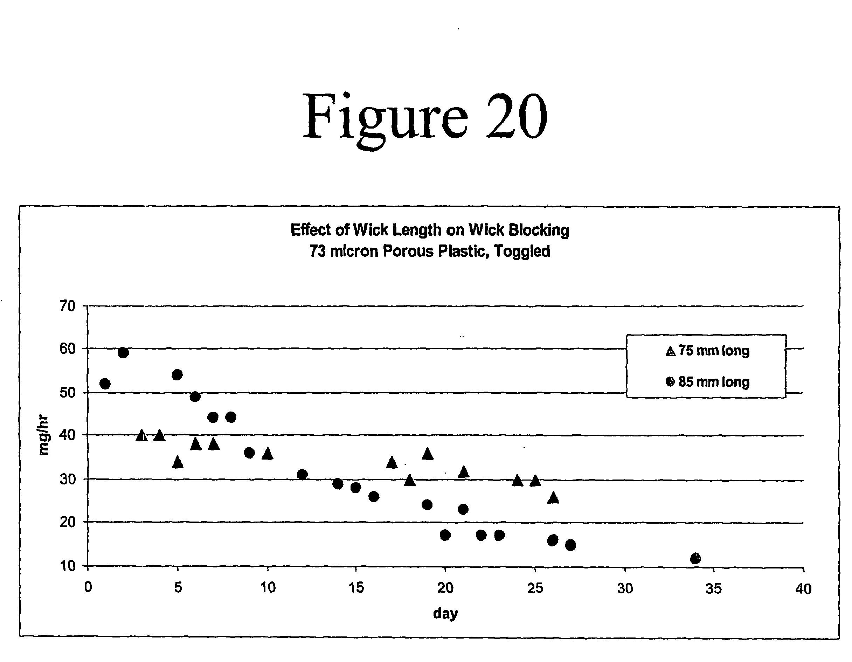

Briefly, an AIR WICK ® Country Berries ® perfume device was tested using two 73-micron wicks: a) 75 mm length, and b) 85 mm length. The two wicks were tested by running a device continuously for more than one month. The device was alternately turned on and off for 72-minute periods. During the "on" portion of each cycle, the heater temperature was 70°C. Figure 20 shows the results.

As can be seen, the shorter wick produced a more stable evaporation profile. After more than three weeks of use, the evaporation had decreased by only about 25%, whereas the evaporation from the longer wick had decreased by more than 50%.

Example 6: Determination of Cycle Time

Additional studies were performed to determine the cycle time that achieved a most desirable scent emission for a two-wick system.

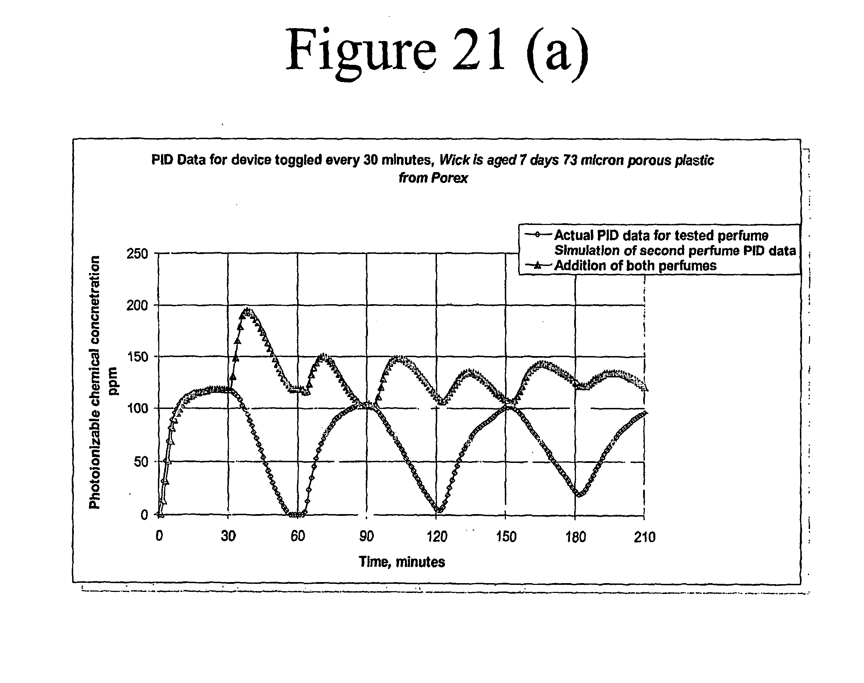

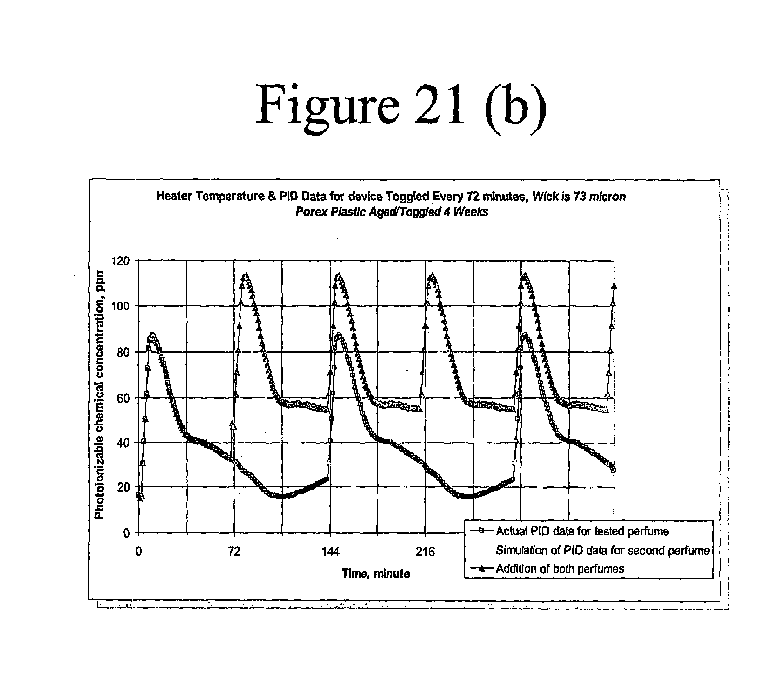

Briefly, two AIR WICK ® Country Berries ® perfume devices were tested. The first was alternately turned on and off for 30-minute periods. The second was alternately turned on and off for 72-minute periods. The emissions were measured using a photoionization detector (PID), Photovac model 2020. The results were plotted versus time. In each case, the recorded results were additionally shifted by 30 and 72 minutes, respectively, to simulate a second, identical, scent-emitting wick. The plots are shown in Figure 21(a) and (b).

As can be seen from the Figure, a 30-minute cycle time for a two-wick system achieved the most desirable level, i.e., the lowest peak:valley ratio for the simulated combined curve. It should be noted, however, that for a 3-wick system, a longer cycle time would be effective. The theoretical ideal is an infinite number of wicks. Obviously, practicality dictates fewer wicks, and 2, 3, 4, or 5 wicks may be more practical.

Example 7: Determination of Impact of Cycle Time on Volatilization

Additional studies were performed to determine the impact of cycle time on volatilization of perfume compositions from a single wick system.

Briefly, four AIR WICK ® Country Berries ® perfumes were used in the test. The wicks were made of porous polyethylene with an average pore size of 73 microns, an average pore volume of 38%, and were 85 mm in length with a diameter of 6.8 mm. During the "on" portion of each cycle, the heater temperature was 70°C and yielded an average wick temperate of 60°C based on temperature measurements at the top (heated portion) of the wick (a temperature probe was placed at the top of the wick.) The first device was alternately turned on and off for 15-minute periods. The second device was alternately turned on and off for 30-minute periods. The third device was alternately turned on and off for 45-minute periods. The fourth device was alternately turned on and off for 72-minute periods. The emissions were measured using a photoionization detector (PID), Photovac model 2020. The test location was a ten by ten foot room with ambient temperature and airflow.

Emissions were tested for each wick after the devices had been in use for two weeks, and then after the devices had been in use for four weeks. A PID sensor was positioned approximately 5 mm above the top center portion of each wick. Emissions were sampled at peak emission (initial emission after heat is applied to the wick) and once per minute throughout three complete on/off cycles. For example, emissions were sampled for the third device at two weeks, once per minute, for a total sample time of 270 minutes; emissions were again sampled for the first device at four weeks, once per minute, for a total sample time of 270 minutes. Electronic readings from the PID were captured and evaluated for average concentration of perfume at the top of the wick in parts per million during the measured periods. Results of one such study are shown in Table 11.

**Table 11 : Impact of Cycle Time on Wick Evaporation: PID Concentration of Chemical at Wick Surface, average PPM/per minute**

| | Wick Age | | | |
|---|---|---|---|---|
| | 2 weeks | | 4 weeks | |
| Toggle time | stabilized | peak | stabilized | peak |
| 15 min | 45 | 50 | | |
| 30 min | 100 | 140 | 55 | 65 |
| 45 min | 95 | 125 | NA | NA |
| 72 min | | | 60 | 110 |

## Claims

1. A method of flattening a pernime-release profile from a heated-wick perfume composition-dispensing device comprising:
a) applying heat to the wick to achieve a wick temperature sufficient to increase the rate of volatilization of at least one component of the perfume composition;
b) reducing the heat to achieve a wick temperature sufficient to decrease the rate of volatilization of the at least one component of the perfume composition;
c) maintaining the reduced heat for a time sufficient to allow for back-flow of at least one component of the perfume composition; and
repeating a).

2. The method according to claim 1, wherein the wick temperature sufficient to increase the rate of volatilization of at least one component of the perfume composition is greater than or equal to 40°C.

3. The method according to claim 2, wherein the wick temperature sufficient to increase the rate of volatilization of at least one component of the perfume composition is greater than or equal to about 60°C.

4. The method according to claim 3, wherein the wick temperature sufficient to increase the rate of volatilization of at least one component of the perfume composition is greater than or equal to 80°C.

5. The method according to claim 1, wherein the wick temperature sufficient to decrease the rate of volatilization of the at least one component of the perfume composition is less than or equal to 40°C.

6. The method according to claim 1, wherein the difference between wick temperatures at a) and c) is from 10 °C to 100 °C.

7. The method according to claim 6, wherein the difference between wick temperatures at a) and c) is from 20 °C to 80 °C.

8. The method according to claim 7, wherein the difference between wick temperatures at a) and c) is from 40 °C to 60°C.

9. The method according to claim 1, wherein the time sufficient to allow for back-flow of all or a portion of the components of the perfume composition is from 15 minutes to 48 hours.

10. The method according to claim 9, wherein the time sufficient to allow for back-flow of all or a portion of the components of the perfume composition is from 17 minutes to 72 minutes.

11. The method according to claim 10, wherein the time sufficient to allow for back-flow of all or a portion of the components of the perfume composition is from 20 minutes to 60 minutes.

12. The method according to claim 11, wherein the time sufficient to allow for back-flow of all or a portion of the components of the perfume composition is 30 minutes.

13. The method according to claim 1, further comprising, repeating b) and o).

14. The method according to claim 13, wherein a), b), and c), are each repeated at least two times.

15. The method according to claim 1, wherein in at least one repeated heating steps, the temperature of the wick is higher than in the previous heating step.

16. The method according to claim 1, wherein the heated-wick perfume composition-dispensing device comprises at least a first and second wick drawing, respectively, from at least a first and second perfume composition reservoir, and the method comprises:
a1) applying heat to the first wick to increase volatilization of at least one component of the first perfume composition;
b1) reducing the heat applied to the first wick to a temperature sufficient to decrease volatilization of the at least one component of the first perfume composition;
c1) maintaining the reduced heat applied to the first wick for a time sufficient to allow for back-flow of all or a portion of the components of the first perfume composition;
a2) applying heat to the second wick to increase volatilization of at least one component of the second perfume composition;
b2) reducing the heat applied to the second wick to a temperature sufficient to decrease volatilization of the at least one component of the second perfume composition;
c2) maintaining the reduced heat applied to the second wick for a time sufficient to allow for back-flow of at least one component of the second perfume composition;
repeating a1); and
repeating a2).

17. The method according to claim 16, wherein performance of a1) and a2) overlaps for a period of from 0.1% to 100% of the duration of a1).

18. The method according to claim 16, wherein the performance of a1) and a2) does not overlap.

19. The method according to claim 18, wherein there is a gap between performance of a1) and a2) for a period of from 0.1% to 100% of the duration of a1).

20. The method according to claim 1, wherein the reduced heat is maintained for a time sufficient to allow for back-flow of all of the components of the perfume composition.

21. method according to Claim 1 wherein the period of emission of a volatile composition is 45 minutes.

22. A scent-dispensing system comprising:
a heated-wick perfume composition-dispensing device that is adapted to receive at least one perfume module, which comprises a reservoir containing a perfume composition, and a wick in fluid communication with said perfume composition,
wherein said device further comprises :
means for applying heat to the wick to increase volatilization of at least one component of the perfume composition;
means for reducing the heat to a temperature sufficient to decrease volatilization of the at least one component of the perfume composition;
means for maintaining the reduced heat for a time sufficient to allow for back-flow of all or a portion of the components of the perfume composition; and
means for applying heat to the wick to increase volatilization of at least one component of the perfume composition, and **characterized in that** the device comprises means to automatically cycle through heat application and heat reduction of each wick, wherein the time to allow for back-flow during each cycle is at least 15 minutes.

23. The scent-dispensing device according to claim 22, wherein the time sufficient to allow for back-flow is at least 30 minutes.

24. The scent-dispensing device according to claim 22, the scent-dispensing device comprises a manually adjustable thermostat.

25. The scent-dispensing system according to claim 22, comprising a means for directing air flow over the wick to enhance evaporation of at least one component of the perfume composition.

26. The scent-dispensing system according to claim 25, wherein said means for directing air flow comprises a vent having slits or louvers or both.

27. The scent-dispensing system according to claim 25, wherein said means for directing air flow comprises a fan.

28. The scent-dispensing system according to claim 25, adapted to receive two or more perfume modules.

29. The scent-dispensing system according to claim 28, comprising at least two wicks, wherein the device, in use, applies heat to each of the wicks in an alternating sequence.

30. The scent-dispensing system according to claim 29, wherein the device further comprises means for applying heat to each of the wicks in a random alternating sequence.

31. The scent-dispensing system according to claim 22, comprising at least two compartments, each compartment being occupied by at least two perfume modules.

32. The scent-dispensing system according to claim 31, comprising a cap that defines at least two vent holes, each vent hole positioned to cover each of said at least two compartments, said cap comprising a movable cover, which further comprises means for being alternately positioned over one or more of each of said vent holes.

33. The scent-dispensing system according to claim 32, wherein at least one of said vent holes is not covered.

34. The scent-dispensing system according to claim 32, wherein the vent holes comprise slits or louvers or both.

35. The scent-dispensing system according to claim 32, comprising a fan for enhancing release of said at least two perfume compositions.

36. The scent-dispensing system according to claim 32, comprising a detection device
wherein said detection device is programmed to turn on said scent dispensing system in response to a stimulus.

37. The scent-dispensing system according to claim 22, wherein said detection device is chosen from motion sensors, light sensors, and noise sensors.

38. The scent-dispensing system according to claim 22, comprising a signal device which is programmed to signal the activation of one or more of at least two perfume composition-dispensing devices.

39. The scent-dispensing system according to claim 38, wherein the signal device is programmed to signal the activation of each of said at least two perfume composition-dispensing devices.

40. The scent-dispeasing system according to claim 38, wherein the signal device emits signals chosen from visual and auditory signals.

41. The scent-dispensing system according to claim 38, wherein said signal device, in use, issues a different signal for each of said at least two perfume composition-dispensing devices.

## Patentansprüche

1. Verfahren zur Verflachung eines Duftstoffabgabeprofils von einer Heizdocht-Duftstoffzusammensetzungsabgabevorrichtung, das Folgendes umfasst:
a) Anlegen von Wärme an den Docht, um eine Dochttemperatur zu erreichen, die ausreicht, um die Verdampfungsrate mindestens einer Komponente der Duftstoffzusammensetzung zu erhöhen;
b) Verringern der Wärme, um eine Dochttemperatur zu erreichen, die ausreicht, um die Verdampfungsrate der mindestens einen Komponente der Duftstoffzusammensetzung zu senken;
c) Beibehalten der verringerten Wärme über eine Zeit, die ausreicht, um einen Rückfluss mindestens einer Komponente der Duftstoffzusammensetzung zu ermöglichen; und
Wiederholen von a).

2. Verfahren nach Anspruch 1, wobei die Dochttemperatur, die ausreicht, um die Verdampfungsrate mindestens einer Komponente der Duftstoffzusammensetzung zu erhöhen, bei oder über 40 °C liegt.

3. Verfahren nach Anspruch 2, wobei die Dochttemperatur, die ausreicht, um die Verdampfungsrate mindestens einer Komponente der Duftstoffzusammensetzung zu erhöhen, bei oder über etwa 60 °C liegt.

4. Verfahren nach Anspruch 3, wobei die Dochttemperatur, die ausreicht, um die Verdampfungsrate mindestens einer Komponente der Duftstoffzusammensetzung zu erhöhen, bei oder über 80 °C liegt.

5. Verfahren nach Anspruch 1, wobei die Dochttemperatur, die ausreicht, um die Verdampfungsrate mindestens einer Komponente der Duftstoffzusammensetzung zu senken, bei oder unter 40 °C liegt.

6. Verfahren nach Anspruch 1, wobei der Unterschied zwischen den Dochttemperaturen in a) und c) 10 °C bis 100 °C beträgt.

7. Verfahren nach Anspruch 6, wobei der Unterschied zwischen den Dochttemperaturen in a) und c) 20 °C bis 80 °C beträgt.

8. Verfahren nach Anspruch 7, wobei der Unterschied zwischen den Dochttemperaturen in a) und c) 40 °C bis 60 °C beträgt.

9. Verfahren nach Anspruch 1, wobei die Zeit, die ausreicht, um ein Zurückfließen aller oder eines Teils der Komponenten der Duftstoffzusammensetzung zu ermöglichen, 15 Minuten bis 48 Stunden beträgt.

10. Verfahren nach Anspruch 9, wobei die Zeit, die ausreicht, um ein Zurückfließen aller oder eines Teils der Komponenten der Duftstoffzusammensetzung zu ermöglichen, 17 Minuten bis 72 Minuten beträgt.

11. Verfahren nach Anspruch 10, wobei die Zeit, die ausreicht, um ein Zurückfließen aller oder eines Teils der Komponenten der Duftstoffzusammensetzung zu ermöglichen, 20 Minuten bis 60 Minuten beträgt.

12. Verfahren nach Anspruch 11, wobei die Zeit, die ausreicht, um ein Zurückfließen aller oder eines Teils der Komponenten der Duftstoffzusammensetzung zu ermöglichen, 30 Minuten beträgt.

13. Verfahren nach Anspruch 1, das ferner das Wiederholen von b) und c) umfasst.

14. Verfahren nach Anspruch 13, wobei a), b) und c) jeweils mindestens zweimal wiederholt werden.

15. Verfahren nach Anspruch 1, wobei in mindestens einem der wiederholten Erwärmungsschritte die Temperatur des Dochts höher ist als im vorhergehenden Erwärmungsschritt.

16. Verfahren nach Anspruch 1, wobei die Heizdocht-Duftstoffzusammensetzungsabgabevorrichtung mindestens einen ersten und einen zweiten Docht, die Duftstoffzusammensetzung aus mindestens einem ersten bzw. einem zweiten Vorratsbehälter aufziehen, umfasst, und wobei das Verfahren Folgendes umfasst:
a1) Anlegen von Wärme an den ersten Docht, um die Verdampfung mindestens einer Komponente der ersten Duftstoffzusammensetzung zu erhöhen;
b1) Verringern der Wärme, die an den ersten Docht angelegt wird, auf eine ausreichende Temperatur, um die Verdampfung der mindestens einen Komponente der ersten Duftstoffzusammensetzung zu senken;
c1) Beibehalten der verringerten Wärme, die an den ersten Docht angelegt wird, über eine Zeit, die ausreicht, um ein Zurückfließen aller oder eines Teils der Komponenten der ersten Duftstoffzusammensetzung zu ermöglichen;
a2) Anlegen von Wärme an den zweiten Docht, um die Verdampfung mindestens einer Komponente der zweiten Duftstoffzusammensetzung zu erhöhen;
b2) Verringern der Wärme, die an den zweiten Docht angelegt wird, auf eine ausreichende Temperatur, um die Verdampfung der mindestens einen Komponente der zweiten Duftstoffzusammensetzung zu senken;
c2) Beibehalten der verringerten Wärme, die an den zweiten Docht angelegt wird, über eine Zeit, die ausreicht, um ein Zurückfließen mindestens einer Komponente der zweiten Duftstoffzusammensetzung zu ermöglichen;
Wiederholen von a1) und
Wiederholen von a2).

17. Verfahren nach Anspruch 16, wobei die Durchführung von a1) und a2) über einen Zeitraum von 0,1 % bis 100 % der Dauer von a1) gleichzeitig stattfindet.

18. Verfahren nach Anspruch 16, wobei die Durchführung von a1) und a) nicht gleichzeitig stattfindet.

19. Verfahren nach Anspruch 18, wobei ein Abstand zwischen der Durchführung von a1) und a2) von einem Zeitraum von 0,1 % bis 100 % der Dauer von a1) vorliegt.

20. Verfahren nach Anspruch 1, wobei die verringerte Wärme über einen Zeitraum beibehalten wird, der ausreicht, um ein Zurückfließen aller Komponenten der Duftstoffzusammensetzung zu ermöglichen.

21. Verfahren nach Anspruch 1, wobei der Zeitraum der Emission einer flüchtigen Zusammensetzung 45 Minuten beträgt.

22. Duftabgabesystem, das Folgendes umfasst:
eine Heizdocht-Duftstoffzusammensetzungsabgabevorrichtung, die mindestens ein Duftstoffmodul aufnehmen kann, das einen Vorratsbehälter, der eine Duftstoffzusammensetzung enthält, sowie einen Docht umfasst, der mit der Duftstoffzusammensetzung in Fluidverbindung steht,
wobei die Vorrichtung ferner Folgendes umfasst:
Mittel zum Anlegen von Wärme an den Docht, um die Verdampfung mindestens einer Komponente der Duftstoffzusammensetzung zu erhöhen;
Mittel zum Verringern der Wärme auf eine Temperatur, die ausreicht, um die Verdampfung mindestens einer Komponente der Duftstoffzusammensetzung zu senken;
Mittel zum Beibehalten der verringerten Wärme über eine Zeit, die ausreicht, um ein Zurückfließen aller oder eines Teils der Komponenten der Duftstoffzusammensetzung zu ermöglichen; und
Mittel zum Anlegen von Wärme an den Docht, um die Verdampfung mindestens einer Komponente der Duftstoffzusammensetzung zu erhöhen, wobei die Vorrichtung Mittel zum automatischen zyklischen Abwechseln zwischen Wärmeanlegung und Wärmereduzierung für jeden Docht umfasst, wobei die Zeit, über die ein Rückfließen während jedes Zyklus möglich ist, mindestens 15 Minuten beträgt.

23. Duftabgabevorrichtung nach Anspruch 22, wobei die Zeit, die ausreicht, um ein Rückfließen zu ermöglichen, mindestens 30 Minuten beträgt.

24. Duftabgabevorrichtung nach Anspruch 22, wobei die Duftabgabevorrichtung einen manuell einstellbaren Thermostat umfasst.

25. Duftabgabesystem nach Anspruch 22, das ein Mittel zur Lenkung eines Luftstroms über den Docht, um die Verdampfung mindestens einer Komponente der Duftstoffzusammensetzung zu verstärken, umfasst.

26. Duftabgabesystem nach Anspruch 25, wobei das Mittel zum Lenken eines Luftstroms eine Lüftung mit Schlitzen oder länglichen Öffnungen oder beidem umfasst.

27. Duftabgabesystem nach Anspruch 25, wobei das Mittel zum Lenken eines Luftstroms ein Gebläse umfasst.

28. Duftabgabesystem nach Anspruch 25, das dafür ausgelegt ist, zwei oder mehr Duftstoffmodule aufzunehmen.

29. Duftabgabesystem nach Anspruch 28, das mindestens zwei Dochte umfasst, wobei die Vorrichtung im Gebrauch Wärme an jeden der Dochte in alternierender Reihenfolge anlegt.

30. Duftabgabesystem nach Anspruch 29, wobei die Vorrichtung ferner Mittel zum Anlegen von Wärme an jeden der Dochte in zufälliger Reihenfolge umfasst.

31. Duftabgabevorrichtung nach Anspruch 22, die mindestens zwei Kammern aufweist, wobei jede Kammer von mindestens zwei Duftstoffmodulen besetzt ist.

32. Duftabgabesystem nach Anspruch 31, das einen Deckel umfasst, der mindestens zwei Lüftungslöcher definiert, wobei die einzelnen Lüftungslöcher so positioniert sind, dass die mindestens zwei Kammern jeweils darunter liegen, wobei der Deckel eine bewegliche Abdeckung umfasst, die ferner Mittel zum abwechselnden Positionieren über einem oder mehreren der einzelnen Lüftungslöcher umfasst.

33. Duftabgabesystem nach Anspruch 32, wobei mindestens eines der Lüftungslöcher nicht abgedeckt ist.

34. Duftabgabesystem nach Anspruch 32, wobei die Lüftungslöcher Schlitze oder längliche Öffnungen oder beides umfassen.

35. Duftabgabesystem nach Anspruch 32, das ein Gebläse zur Verstärkung der Abgabe der mindestens zwei Duftstoffzusammensetzungen umfasst.

36. Duftabgabesystem nach Anspruch 32, das eine Erfassungseinrichtung umfasst, wobei die Erfassungseinrichtung so programmiert ist, dass sie das Duftabgabesystem auf einen Stimulus hin einschaltet.

37. Duftabgabesystem nach Anspruch 22, wobei die Erfassungseinrichtung aus Bewegungssensoren, Lichtsensoren und Geräuschsensoren ausgewählt ist.

38. Duftabgabesystem nach Anspruch 22, das eine Signaleinrichtung umfasst, die so programmiert ist, dass sie die Aktivierung einer oder mehrerer von mindestens zwei Duftstoffzusammensetzungsabgabevorrichtungen signalisiert.

39. Duftabgabesystem nach Anspruch 38, wobei die Signaleinrichtung so programmiert ist, dass sie die Aktivierung jeder der mindestens zwei Duftstoffzusammensetzungsabgabevorrichtungen signalisiert.

40. Duftabgabesystem nach Anspruch 38, wobei die Signaleinrichtung Signale ausgibt, die aus optischen und akustischen Signalen ausgewählt sind.

41. Duftabgabesystem nach Anspruch 38, wobei die Signaleinrichtung im Gebrauch für jede der mindestens zwei Duftstoffzusammensetzungsabgabevorrichtungen ein anderes Signal ausgibt.

## Revendications

1. Procédé d'aplatissement d'un profil de libération de parfum à partir d'un dispositif dispensateur d'une composition parfumée par mèche chauffée comprenant :
a) l'application de chaleur sur la mèche pour atteindre une température de mèche suffisante pour augmenter le taux de volatilisation d'au moins un composant de la composition parfumée ;
b) la réduction de la chaleur pour atteindre une température de mèche suffisante pour diminuer le taux de volatilisation du au moins un composant de la composition parfumée ;
c) le maintien de la chaleur réduite pendant un temps suffisant pour permettre le reflux d'au moins un composant de la composition parfumée ; et
la répétition de a).

2. Procédé selon la revendication 1, dans lequel la température de la mèche suffisante pour augmenter le taux de volatilisation d'au moins un composant de la composition parfumée est supérieure ou égale à 40 °C.

3. Procédé selon la revendication 2, dans lequel la température de la mèche suffisante pour augmenter le taux de volatilisation d'au moins un composant de la composition parfumée est supérieure ou égale à environ 60 °C.

4. Procédé selon la revendication 3, dans lequel la température de la mèche suffisante pour augmenter le taux de volatilisation d'au moins un composant de la composition parfumée est supérieure ou égale à 80 °C.

5. Procédé selon la revendication 1, dans lequel la température de la mèche suffisante pour diminuer le taux de volatilisation du au moins un composant de la composition parfumée est inférieure ou égale à 40 °C.

6. Procédé selon la revendication 1, dans lequel la différence entre les températures des mèches de a) et c) va de 10 °C à 100 °C.

7. Procédé selon la revendication 6, dans lequel la différence entre les températures des mèches de a) et c) va de 20 °C à 80 °C.

8. Procédé selon la revendication 7, dans lequel la différence entre les températures des mèches de a) et c) va de 40 °C à 60 °C.

9. Procédé selon la revendication 1, dans lequel le temps suffisant pour permettre le reflux de tous les ou d'une partie des composants de la composition parfumée va de 15 minutes à 48 heures.

10. Procédé selon la revendication 9, dans lequel le temps suffisant pour permettre le reflux de tous les ou d'une partie des composants de la composition parfumée va de 17 minutes à 72 minutes.

11. Procédé selon la revendication 10, dans lequel le temps suffisant pour permettre le reflux de tous les ou d'une partie des composants de la composition parfumée va de 20 minutes à 60 minutes.

12. Procédé selon la revendication 11, dans lequel le temps suffisant pour permettre le reflux de tous les ou d'une partie des composants de la composition parfumée est de 30 minutes.

13. Procédé selon la revendication 1, comprenant, en outre, la répétition de b) et de c).

14. Procédé selon la revendication 13, dans lequel a), b), et c), sont chacun répétés au moins deux fois.

15. Procédé selon la revendication 1, dans lequel dans au moins une des étapes de chauffage répété, la température de la mèche est supérieure par rapport à l'étape de chauffage précédente.

16. Procédé selon la revendication 1, dans lequel le dispositif dispensateur d'une composition parfumée par mèche chauffée comprend au moins un premier et un deuxième étirage de mèche, respectivement, depuis au moins un premier et un deuxième réservoir de composition parfumée, et le procédé comprend :
a1) l'application de chaleur sur la première mèche pour augmenter la volatilisation d'au moins un composant de la première composition parfumée ;
b1) la réduction de la chaleur appliquée sur la première mèche à une température suffisante pour diminuer la volatilisation du au moins un composant de la première composition parfumée ;
c1) le maintien de la chaleur réduite appliquée sur la première mèche pendant un temps suffisant pour permettre le reflux de tous les ou d'une partie des composants de la première composition parfumée ;
a2) l'application de chaleur sur la deuxième mèche pour augmenter la volatilisation du au moins un composant de la deuxième composition parfumée ;
b2) la réduction de la chaleur appliquée sur la deuxième mèche à une température suffisante pour diminuer la volatilisation du au moins un composant de la deuxième composition parfumée ;
c2) le maintien de la chaleur réduite appliquée sur la deuxième mèche pendant un temps suffisant pour permettre le reflux d'au moins un composant de la deuxième composition parfumée ;
la répétition de a1) ; et
la répétition de a2).

17. Procédé selon la revendication 16, dans lequel l'exécution de a1) et a2) se chevauche pendant une période comprise entre 0,1 % et 100 % de la durée de a1).

18. Procédé selon la revendication 16, dans lequel l'exécution de a1) et de a2) ne se chevauche pas.

19. Procédé selon la revendication 18, dans lequel il y a un trou entre l'exécution de a1) et de a2) pendant une période comprise entre 0,1 % et 100 % de la durée de a1).

20. Procédé selon la revendication 1, dans lequel la chaleur réduite est maintenue pendant un temps suffisant pour permettre le reflux de tous les composants de la composition parfumée.

21. Procédé selon la revendication 1, dans lequel la période d'émission d'une composition volatile est de 45 minutes.

22. Système de distribution d'une senteur comprenant :
un dispositif dispensateur d'une composition parfumée par mèche chauffée qui est adapté pour recevoir au moins un module de parfum, qui comprend un réservoir contenant une composition parfumée, et une mèche en communication du point de vue des fluides avec ladite composition parfumée,
dans lequel ledit dispositif, comprend, en outre :
un moyen pour appliquer la chaleur sur la mèche pour augmenter la volatilisation d'au moins un composant de la composition parfumée ;
un moyen pour réduire la chaleur à une température suffisante pour diminuer la volatilisation du au moins un composant de la composition parfumée ;
un moyen pour maintenir la chaleur réduite pendant un temps suffisant pour permettre le reflux de tous les ou d'une partie des composants de la composition parfumée ; et
un moyen pour appliquer la chaleur sur la mèche pour augmenter la volatilisation d'au moins un composant de la composition parfumée, et le dispositif comprend un moyen de cycle automatique à travers l'application de chaleur et la réduction de chaleur de chaque mèche, dans lequel le temps pour permettre le reflux pendant chaque cycle est au moins 15 minutes.

23. Dispositif dispensateur de senteur selon la revendication 22, dans lequel le temps suffisant pour permettre le reflux est d'au moins 30 minutes.

24. Dispositif dispensateur de senteur selon la revendication 22, dans lequel le dispositif dispensateur de senteur comprend un thermostat ajustable manuellement.

25. Système dispensateur de senteur selon la revendication 22, comprenant un moyen pour diriger le débit d'air sur la mèche pour renforcer l'évaporation d'au moins un composant de la composition parfumée.

26. Système dispensateur de senteur selon la revendication 25, dans lequel ledit moyen pour diriger le débit d'air comprend une mise à l'air ayant des fentes ou des volets ou l'un et l'autre.

27. Système dispensateur de senteur selon la revendication 25, dans lequel ledit moyen pour diriger le débit d'air comprend un ventilateur.

28. Système dispensateur de senteur selon la revendication 25, adapté pour recevoir deux ou plusieurs modules de parfum.

29. Système dispensateur de senteur selon la revendication 28, comprenant au moins deux mèches, dans lequel le dispositif, en cours d'utilisation, applique de la chaleur sur chacune des mèches dans un ordre alternatif.

30. Système dispensateur de senteur selon la revendication 29, dans lequel le dispositif, comprend, en outre, un moyen pour appliquer de la chaleur sur chacune des mèches dans un ordre alternatif aléatoire.

31. Système dispensateur de senteur selon la revendication 22, comprenant au moins deux compartiments, chaque compartiment étant occupé par au moins deux modules de parfum.

32. Système dispensateur de senteur selon la revendication 31, comprenant une coiffe qui définit au moins deux orifices de mise à l'air, chaque orifice de mise à l'air positionné pour protéger chacun desdits au moins deux compartiments, ladite coiffe comprenant une protection mobile, qui comprend, en outre, un moyen pour être alternativement positionnée sur un ou plusieurs de chacun desdits orifices de mise à l'air.

33. Système dispensateur de senteur selon la revendication 32, dans lequel au moins un desdits orifices de mise à l'air n'est pas couvert.

34. Système dispensateur de senteur selon la revendication 32, dans lequel les orifices de mise à l'air comprennent des fentes ou des volets ou les deux.

35. Système dispensateur de senteur selon la revendication 32, comprenant un ventilateur pour renforcer la libération desdites au moins deux compositions parfumées.

36. Système dispensateur de senteur selon la revendication 32, comprenant un dispositif de détection où ledit dispositif de détection est programmé pour allumer ledit système dispensateur de senteur en réponse à une stimulation.

37. Système dispensateur de senteur selon la revendication 22, dans lequel ledit dispositif de détection est choisi parmi des capteurs de mouvement, des capteurs de lumière, et des capteurs de bruit.

38. Système dispensateur de senteur selon la revendication 22, comprenant un dispositif de signal qui est programmé pour signaler l'activation d'un ou plusieurs des au moins deux dispositifs dispensateurs de composition parfumée.

39. Système dispensateur de senteur selon la revendication 38, dans lequel le dispositif de signal est programmé pour signaler l'activation de chacun desdits au moins deux dispositifs dispensateurs de composition parfumée.

40. Système dispensateur de senteur selon la revendication 38, dans lequel le dispositif de signal émet des signaux choisis parmi des signaux auditifs et visuels.

41. Système dispensateur de senteur selon la revendication 38, dans lequel ledit dispositif de signal, en cours d'utilisation, fournit un signal différent pour chacun desdits au moins deux dispositifs dispensateurs de composition parfumée.
